(19) Europäisches Patentamt
European Patent Office
Office européen des brevets

(11) EP 2 403 953 B1

(12) **EUROPÄISCHE PATENTSCHRIFT**

(45) Veröffentlichungstag und Bekanntmachung des Hinweises auf die Patenterteilung:
**10.05.2017 Patentblatt 2017/19**

(21) Anmeldenummer: **10706255.6**

(22) Anmeldetag: **03.03.2010**

(51) Int Cl.:
*C12P 7/06* (2006.01) *C12P 7/16* (2006.01)
*C12P 7/18* (2006.01) *C12P 7/22* (2006.01)
*C12P 7/42* (2006.01) *C12N 9/02* (2006.01)
*C12P 7/02* (2006.01) *C12P 7/24* (2006.01)
*C12P 41/00* (2006.01)

(86) Internationale Anmeldenummer:
**PCT/EP2010/052701**

(87) Internationale Veröffentlichungsnummer:
**WO 2010/100195 (10.09.2010 Gazette 2010/36)**

(54) **VERFAHREN ZUR STEREOSELEKTIVEN ENZYMATISCHEN REDUKTION VON KETOVERBINDUNGEN**

METHOD FOR STEREOSELECTIVE ENZYMATIC REDUCTION OF KETO COMPOUNDS

PROCÉDÉ DE RÉDUCTION STÉRÉOSÉLECTIVE ENZYMATIQUE DE COMPOSÉS CÉTONIQUES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **05.03.2009 EP 09450050**

(43) Veröffentlichungstag der Anmeldung:
**11.01.2012 Patentblatt 2012/02**

(73) Patentinhaber: **Cambrex IEP GmbH**
**65203 Wiesbaden (DE)**

(72) Erfinder:
- **GUPTA, Antje**
  **65207 Wiesbaden (DE)**
- **DUPONT, Maria**
  **50354 Hürth (DE)**
- **TSCHENTSCHER, Anke**
  **65347 Eltville - Hattenheim (DE)**

(74) Vertreter: **Plate, Jürgen et al**
**Plate Schweitzer Zounek**
**Patentanwälte**
**Rheingaustrasse 196**
**65203 Wiesbaden (DE)**

(56) Entgegenhaltungen:
**WO-A-02/12544** **WO-A-02/086126**
**WO-A-2006/087235** **WO-A-2007/012428**
**WO-A-2007/073875**

- **DATABASE UniProt [Online] 1. Juni 2003 (2003-06-01), "SubName: Full=Short chain dehydrogenase;" XP002541641 gefunden im EBI accession no. UNIPROT:Q81AU6 Database accession no. Q81AU6**
- **DATABASE UniProt [Online] 22. August 2006 (2006-08-22), "SubName: Full=Short-chain dehydrogenase/reductase SDR; Flags: Precursor;" XP002541642 gefunden im EBI accession no. UNIPROT:Q11G59 Database accession no. Q11G59**
- **JORNVALL H ET AL: "SDR and MDR: completed genome sequences show these protein families to be large, of old origin, and of complex nature" FEBS LETTERS, ELSEVIER, AMSTERDAM, NL, Bd. 445, Nr. 2-3, 26. Februar 1999 (1999-02-26), Seiten 261-264, XP004259268 ISSN: 0014-5793**
- **OPPERMANN U C T ET AL: "STRUCTURE-FUNCTION RELATIONSHIPS OF SDR HYDROXYSTEROID DEHYDROGENASES" ADVANCES IN EXPERIMENTAL MEDICINE AND BIOLOGY, SPRINGER, US, Bd. 414, 1. Januar 1997 (1997-01-01), Seiten 403-415, XP008012012 ISSN: 0065-2598**
- **JOERNWALL H ET AL: "SHORT-CHAIN DEHYDROGENASES/REDUCTASES (SDR)" BIOCHEMISTRY, AMERICAN CHEMICAL SOCIETY, EASTON, PA.; US, Bd. 34, Nr. 18, 9. Mai 1995 (1995-05-09), Seiten 6003-6013, XP000929805 ISSN: 0006-2960**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 403 953 B1

## Beschreibung

Die vorliegende Erfindung betrifft ein Verfahren zur stereoselektiven, insbesondere enantioselektiven enzymatischen Reduktion von Ketoverbindungen zu den entsprechenden chiralen Hydroxyverbindungen, wobei die Ketoverbindungen mit einer enantioselektiven, NADH-spezifischen Oxidoreduktase reduziert werden. Die Erfindung betrifft insbesondere die Verwendung ausgewählter Oxidoreduktasen in diesem Verfahren.

Optisch aktive Hydroxyverbindungen sind wertvolle chirale Bausteine mit breiter Anwendung für die Synthese von pharmakologisch wirksamen Verbindungen, aromatischen Substanzen, Pheromonen, Agrochemikalien und Enzyminhibitoren. Dabei ist insbesondere im Pharmasektor ein steigender Bedarf an chiralen Verbindungen und somit chiralen Synthesetechnologien zu verzeichnen, da racemische Verbindungen in Zukunft kaum noch als Arzneimittel Verwendung finden werden.

Die asymmetrische Reduktion prochiraler Ketoverbindungen ist ein Sektor der stereoselektiven Katalyse, in dem die Biokatalyse eine leistungsfähige Konkurrenztechnologie zur chemischen Katalyse darstellt. Die chemische asymmetrische Hydrierung erfordert den Einsatz hochgiftiger und umweltbelastender Schwermetallkatalysatoren, extremer und somit energieintensiver Reaktionsbedingungen sowie große Mengen organischer Lösungsmittel. Desweiteren sind diese Verfahren häufig gekennzeichnet durch Nebenreaktionen und unzureichende Enantiomerenüberschüsse.

Reduktionen prochiraler Ketoverbindungen zu Hydroxyverbindungen und umgekehrt ablaufende Oxidationen kommen in der Natur in zahlreichen biochemischen Pathways, sowohl im Primärstoffwechsel als auch im Sekundärstoffwechsel, in jedem Organismus vor und werden von unterschiedlichen Typen von sekundären Alkoholdehydrogenasen (ADH) und Oxidoreduktasen katalysiert. Diese Enzyme sind in der Regel cofaktorabhängig.

In verschiedenen Sequenzierungsprojekten wurden für unterschiedliche Organismen jeweils zahlreiche Aminosäuresequenzen identifiziert, bei denen es sich vermutlich um Oxidoreduktasen unbekannter Aktivität, Funktion und Enantio- bzw. Chemoselektivität handelt. Die grundsätzliche Eignung von Oxidoreduktasen zur Anwendung in industriellen Prozessen konnte jüngst anhand zahlreicher Beispiele demonstriert werden.

Vor kurzem konnte gezeigt werden, dass die Verwendung isolierter Oxidoreduktasen in wässrig/organischen Zwei-Phasen-Systemen mit organischen Lösungsmitteln äußerst effizient, ökonomisch und auch in hohen Konzentrationen (> 5%) möglich ist. Bei den beschriebenen Systemen bildet dabei die zu reduzierende, meist schlecht wasserlösliche Ketoverbindung zusammen mit dem organischen Lösungsmittel die organische Phase. Teilweise kann auch auf das organische Lösungsmittel selbst verzichtet werden. In diesem Fall wird dann die organische Phase von der zu reduzierenden Ketoverbindung gebildet (EP 1 383 899). Die Coenzymregenerierung wird durch die gleichzeitige Oxidation sekundärer Alkohole realisiert, wobei in den meisten Fällen das preiswerte wassermischbare 2-Propanol verwendet wird (WO 2006/087235). Ebenfalls vorteilhaft ist der Einsatz von 2-Methyl-4-pentanol oder 2-Oktanol zur Regenerierung der Cofaktoren NADH oder NADPH (WO 2007/036257).

In der Patentliteratur der letzten Jahre finden sich zahlreiche Oxidoreduktasen unterschiedlicher Spezifiät und Selektivität. Verschiedene Beispiele findet man für S-spezifische Oxidoreduktasen und Dehydrogenasen hoher Enantioselektivität, welche fast ausnahmslos NADH als Cofaktor verwenden und daher sehr ökonomisch unter oben beschriebenen Prozessbedingungen eingesetzt werden können. Beispiele für solche S-spezifische Oxidoreduktasen sind die Carbonylreduktasen aus *Candida parapsilosis* (CPCR) (US 5,523,223 und US 5,763,236; Enzyme Microb. Technol. 1993 Nov; 15(11):950-8) und *Pichia capsulata* (DE 10327454.4), Carbonylreduktasen aus *Rhodococcus erythropolis* (RECR) (US 5,523,223), *Norcardia fusca* (Biosci. Biotechnol. Biochem., 63(10) (1999), S. 1721-1729; Appl. Microbiol. Biotechnol. 2003 Sept; 62(4):380-6; Epub 2003 Apr 26) und *Rhodococcus ruber* (J. Org. Chem. 2003 Jan 24; 68(2):402-6). Ferner beschrieben sind S-spezifische Carbonylreduktasen aus *Rhodotorula mucillaginosa*, *Microbacterium spec*., *Gordonia rubripertincta*, *Pichia stipitis* und *Pichia trehalophila* (WO 2007/012428).

Beispiele für R-spezifische sekundäre Alkoholdehydrogenasen sind beschrieben aus Organismen der Gattung *Lactobacillus*: *Lactobacillus kefir* (US 5,200,335), *Lactobacillus brevis* (DE 19610984 A1; Acta Crystallogr. D Biol. Crystallogr. 2000 Dec; 56 Pt 12:1696-8*)*, *Lactobacillus minor* (DE 10119274) und *Leuconostoc carnosum* (WO 2007/012428). Diese Enzyme haben alle den Nachteil, dass als Cofaktor NADPH benötigt wird, welches wesentlich teurer ist als NADH und somit verhältnismäßig hohe Prozesskosten impliziert.

Unter "R-spezifischen" Oxidoreduktasen werden solche verstanden, die unsubstituierte Carbonylverbindungen, wie beispielsweise 2-Butanon, 2-Oktanon oder Acetophenon, zu den entsprechenden R-Hydroxyverbindungen, also R-2-Butanol, R-2-Oktanol und R-2-Phenylethanol, reduzieren. 4-Halo-3-oxobuttersäureester beispielsweise wird von R-spezifischen Oxidoreduktasen zu S-4-Halo-3-hydroxybuttersäureester reduziert.

"S-spezifische Oxidoreduktasen " sind solche ADH, die unsubstituierte Carbonylverbindungen, wie beispielsweise 2-Butanon, 2-Oktanon oder Acetophenon, zu den entsprechenden S-Hydroxyverbindungen, also S-2-Butanol, S-2-Oktanol und S-2-Phenylethanol, reduzieren. 4-Halo-3-oxobuttersäureester beispielsweise wird von S-spezifischen Oxidoreduktasen zu R-4-Halo-3-hydroxybuttersäureester reduziert.

Unter dem Begriff "spezifisch" ist in der vorliegenden Anmeldung zu verstehen, dass das entsprechende Enantiomer zu >95% gebildet wird.

Desweiteren ist in der jüngsten Patentliteratur eine Reihe von unspezifischen NADPH-abhängigen Enzymen aus Hefen der Gattung *Candida magnoliae* beschrieben (EP 1152054 A1, WO 2007/033928, WO 2006/046455, WO 2005/033094). Diese haben neben der NADPH-Abhängigkeit den großen Nachteil, dass sie nicht in Verfahren mit substratgekoppelter Coenzymregenerierung verwendet werden können, sondern stets zur Coenzymregenerierung ein zweites Enzymsystem benötigen. Bevorzugt wird hierzu Glucosedehydrogenase mit Glucose als Cosubstrat verwendet. Außerdem haben diese Enzyme den Nachteil, dass sie in der Regel unspezifisch sind, d.h die Reduktion unsubstituierter Carbonylverbindungen, wie beispielsweise 2-Butanon, 2-Oktanon oder Acetophenon, führt in der Regel zu racemischen Alkoholen; nur in Ausnahmefälle arbeiten diese Enzyme selektiv. Daher ist die enatioselektive Reduktion von Substraten, wie 4-Halo-3-oxobuttersäureester, 3-Oxoestern oder aliphatischen Ketonen, z.B. 2-Oktanon, mit diesen Enzymen in der Regel nicht möglich.

Nur eine sehr begrenzte Anzahl robuster, NADH-abhängiger und deutlich R-spezifischer Oxidoreduktasen, die in Prozessen mit substratgekoppelter Coenzymregenerierung mit sekundären Alkoholen, bevorzugt 2-Propanol, als Cosubstrat einsetzbar sind, steht zur Verfügung.

Eine NADH-abhängige R-spezifische Oxidoreduktase ist beispielsweise beschrieben aus *Pichia finlandica* (EP 1179 595 A1). Diese weist aber eine sehr geringe Aktivität mit 2-Propanol auf und eignet sich dadurch nicht für Prozesse mit substratgekoppelter Coenzymregenerierung.

Zwei weitere NADH-abhängige R-spezifische Oxidoreduktasen aus *Pichia farinosa* und *Candida nemodendra* sind in der WO 2007/012428 beschrieben. Darin konnte für das Enzym aus *Pichia farinosa* gezeigt werden, dass auch eine Coenzymregeneration mit 2-Propanol möglich ist, allerdings nur in Konzentrationen bis zu 15% (v/v) Isopropanol.

Weiters bekannt sind NADH-abhängige Enzyme aus *Leifsonia* (US 7,172,894 B2, Biosci. Biotechnol. Biochem.,70 (2),2006, pages 418-426) und *Devosia* (WO 2004/027055), wobei zumindest das Enzym aus *Leifsonia* R-spezifisch und zur substratgekoppelten Coenzymregenerierung befähigt sein sollte.

Die heute bekannten Oxidoreduktasen, insbesondere die eindeutig R-spezifischen Enzyme, reichen aber bei weitem nicht aus, um das gesamte Marktpotential an stereoselektiven Reduktionen von Ketoverbindungen auszuschöpfen. Das ist einerseits damit zu begründen, dass die einzelnen Enzyme über sehr unterschiedliche Eigenschaften bezüglich Substratspektrum, pH-Optimum sowie Temperatur- und Lösungsmittelstabilitäten verfügen, die sich häufig gegenseitig ergänzen. Daher können selbst verhältnismäßig ähnliche, homologe Enzyme im Hinblick auf ein bestimmtes Substrat ein völlig unterschiedliches Umsetzungsverhalten aufweisen. Andererseits sind längst nicht alle beschriebenen Enzyme kloniert und in ausreichendem Maße überexprimierbar, was bedeutet, dass diese Enzyme für eine industrielle Anwendung nicht zur Verfügung stehen.

Daher ist es zur möglichst breiten Ausschöpfung des synthetischen Potentials der enzymatischen asymmetrischen Hydrierung notwendig, über ein möglichst breites Portfolio industriell zugänglicher und unterschiedlicher Oxidoreduktasen zu verfügen. Mittlerweile können die Anforderungen an Enzyme, die vorteilhaft industriell einsetzbar sind, klar definiert werden.

Ziel der vorliegenden Erfindung ist daher die Identfizierung und die Bereitstellung stabiler, NADH-abhängiger und R-spezifischer Oxidoreduktasen, welche für Verfahren zur Reduktion von Ketoverbindungen zu chiralen Alkoholen unter Verwendung von substratgekoppelter Coenzymregenerierung geeignet sind.

Hierzu ist die Bereitstellung von Oxidoreduktasen mit möglichst hoher Stabilität gegenüber sekundären Alkoholen, insbesondere gegenüber Isopropanol, erforderlich. Desweiteren sollten sich die bereitzustellenden Enzyme durch gute Exprimierbarkeit in *Escherichia coli* (>500 Units/g *E. coli* Biofeuchtmasse) auszeichnen.

Aufgrund der hohen Anforderung an Stabilität und Exprimierbarkeit in E. coli wurde in der vorliegenden Erfindung davon ausgegangen, dass bakterielle Enzyme gegenüber Enzymen aus Hefe zu bevorzugen sind.

Ziel der vorliegenden Erfindung ist ferner die Identifizierung solcher Aminosäuresequenzen bzw. Gensequenzen, die rekombinant exprimiert stabile NADH-abhängige, (R)-spezifische Oxidoreduktasen darstellen und sich unter Verwendung enzymgekoppelter Coenzymregenerierung prozesstechnisch eignen.

Es wurde von den Erfindern festgestellt, dass solche Sequenzen allgemein die bekannten Merkmale einer "short-chain"-Dehydrogenase tragen, wie beispielsweise einen N-terminalen Rossmann-Fold GxxxGxG für die Cofaktorbindung, ein NAG-Motiv an der Position 87, das für Stabilisierung der zentralen ß-Faltblatt-Struktur sorgt, und eine hochkonservierte katalytische Triade, bestehend aus S 139, Y 152 und K 156 im aktiven Zentrum. Für die Zwecke der vorliegenden Anmeldung erfolgt die nummerische Zuordnung einzelner homologer Aminosäurereste in den Proteinsequenzen durch den "multi-way protein alignment algorithm" mit Score Matrix BLOSUM62 mit der Referenzsequenz 2bhd_Strex und den Aminosäuresequenzen SEQ ID NO1 bis SEQ ID NO:7.

Bei der Vielfalt an im Internet verfügbaren Aminosäurensequenzen, die diese Signatur aufweisen, bedurfte es der Definition weiterer Merkmale, um diesen Sequenzpool einzuschränken. Von den Erfindern wurde daher postuliert, dass (R)-spezifische Oxidoreduktasen mit den beschriebenen Eigenschaften zusätzlich folgende Sequenzmerkmale und Muster aufweisen müssen:

- einen negativ geladenen Aminosäurerest an Position 37
- zwei C-terminale Motive in der Dimerisierungsdomäne: [A; S]-S-F und [V; I]-DG-[G;A]-Y-[T; C; L]-[A; T; S]-[Q; V; R; L; P]
- eine R-ADH-Signatur an der Position205 bis 209: H-[P; A]-[I; A; Q; V; L]-[G; K]-R
- Val oder Leu an der Position 159
- Asn an der Position 178
- einen Prolinrest an der Position 188.

Nach diesem Muster wurden aus dem aus Sequenzierungsprojekten zugänglichen Sequenzpool folgende Sequenzen ohne vorhandene Funktionszuordnung ausgewählt, kloniert und hinsichtlich ihrer Funktion charakterisiert.

**Tabelle 1**.

| SEQ ID NO | Ursprung | Länge | Zugangsnummer | Nächste Homologe |
|---|---|---|---|---|
| SEQ ID NO:1 | Bacillus cereus ATCC 14579 | 247 aa | NP-833194 | Glucose-1-Dehydrogenase aus Lysinibacillus 59% Identität |
| SEQ ID NO:2 | Methylibium petroleiphilum PmI | 257 aa | YP-001020081 | Short-chain Dehydrogenase Flavobakterium 59% Identität |
| SEQ ID NO:3 | Mesorhizobium sp. | 251 aa | YP-676781 | Putative Oxidoreduktase Sinorhizobium 62% Identität |
| SEQ ID NO:4 | Streptomyces coelicolor | 263 aa | NP-631416 | short-chain Dehydrogenase Comamonas testosteroni 57% Identität |
| SEQ ID NO:5 | Ralstonia | 253 aa | YP-299653 (epimerase/dehydratase/ reductase SDR) | short-chain Dehydrogenase Bacillus 54% Identität |
| SEO ID NO:6 | Herpentosiphon aurantiacus ATCC 23779 | 252 aa | YP-001544828 | short-chain Dehydrogenase Comamonas testosteroni 54% Identität |
| SEQ ID NO:7 | Paracoccus denitrificans | 249 aa | ZP-00631061 | short-chain Dehydrogenase, Pedobacter 57% Identität |

```
2bhd_Strex     1 -----------mhdltgkn--viitggarglgaeaarqavaagahvlitd
Seq ID No 1    1 --------------mklkdkvaiitggasgigestvrlfieegakvviad
Seq ID No 2    1 ---------mgrvd----nkvalvtggakgigrasalmlaregarvvltd
Seq ID No 3    1 ------------mtgefkdkvalvtgagsgigaaiarelatggaelvvad
Seq ID No 4    1 msttgttpattgyaaefagrtalvtgaasgiglatarrlgaggarvvvad
Seq ID No 5    1 ---------meyidmklkdkvaivtggasgigeatvrlfasqgasvviad
Seq ID No 6    1 -----------mnqydfrgkvalvtggasgigaacvhtfarggakvaivd
Seq ID No 7    1 ------------mdirfdnkialvtgagsglgeaialelaasgatvvaad

2bhd_Strex    38 vldddg-enaarel---gdrarflhhdvtseedwsraadfavtefgalhg
Seq ID No 1   37 fser--gkelsdelnahgyntlfiktdvtkeadikqlihetvstygkldi
Seq ID No 2   38 veeaqgsa-vakeieraggkalfltqdvtdesrwvevvekaraqfgglni
Seq ID No 3   39 lere-sanrivegirasggrahafavdvadaeavermvdfavrtcgdlhl
Seq ID No 4   51 fnae-gaekaaaelraggveaaaveldvtrpesveaavgfavdtfgsldl
Seq ID No 5   42 rsal--geklarelsesslaahysevdvsreddtrrliddtvsrfgrldi
Seq ID No 6   40 rnqdlgaqtvaa-vreaggdaiflpvdvaqsgaveamvtdtitafgqldi
Seq ID No 7   39 lhea-taratadrivaaggkakavagdvsdpdavrkaveva-kglgglhl

2bhd_Strex    84 lvnnagis-tgtplesesvdhfrkvldvnltgvfigmktvvpa-ikeagg
Seq ID No 1   85 myanagvad-dapanelsyekwkrtidinlsgvflsdkysieqfikqgtg
Seq ID No 2   87 vvnnagig-tagsaedetleawrrlmsvnldgvflgtkhairam-kngpg
Seq ID No 3   88 avnnagiggpseptadypldgwrkvidvnlngvfygmkyeiaailksg-g
Seq ID No 4  100 avnnagiggpsaptgeydvaayqrvvrtnldgvfysmryelpaieaagkg
Seq ID No 5   90 mvanagiahpsapvedvsveqwqqmidvnltgvflsnklaivqmkkqgtg
Seq ID No 6   89 avnnagiggesnptgtysiegwqtvidvnlngvfycmryeipamlaqg-g
Seq ID No 7   87 lvnnagiggpsapvgdypldgwkkvidvnlnavfygmrygipamldag-g

2bhd_Strex   132 -gsivnissaaglmglaltagygaskwgvrgltkigavewgta--rvrvn
Seq ID No 1  134 -gvivnagsihsfvslptptayssakggvklltqnlctayakyg--irin
Seq ID No 2  135 tgsiinissiegivadpklasynaskggvrifsksaalhcaqagyriirvn
Seq ID No 3  137 -gaivnmasilgsvgfanacayvsakhallgltktaameyaaqg--vrin
Seq ID No 4  150 -gsivnvasilgsvgfagspayvaakhgvvgltkaaaaeyaarg--irin
Seq ID No 5  140 -gaivnmasilghvgmpgaasynaakggvvnltrslgvshaqdg--irvn
Seq ID No 6  138 -gvivnmasilgtvgfasspayvaakhavvgltkaaaldygrqg--lrin
Seq ID No 7  136 -gaivnmasilgsvgfngagayvsakhavvgmtknaaleyaqkg--irvn

2bhd_Strex   179 svhpgmtytpmtaavgiergegkypnt------pmgrvgeadeiagavvf
Seq ID No 1  181 avcpgyidtpllgsv-n-----pqqkeylaslhpqgrlgtpeevakavlf
Seq ID No 2  185 tihpgyiwtpmvegyltslgdveggrqviskmhpigrmgepddiaygvly
Seq ID No 3  184 avgpafidtpllsknld-----dqvlgqlaglhpigrlgtpeevsaltcf
Seq ID No 4  197 avgpgfidtpll-ktmd-----eaaykglvalhpagrlgrseevaeliaf
Seq ID No 5  187 avcpgfvatpliera-t-----eeararlvaahpigrlghadevakavlf
Seq ID No 6  185 svgpgfiktplldggld-----dqtqtylsglhavgrmgesaevaalvaf
Seq ID No 7  183 svgpafidtplldq-ld-----sdtrqalvgrhpigrlgradevaglvvf

2bhd_Strex   223 llsdaasyvtgaelavdggwttgptvayvmgq
Seq ID No 1  225 lasddasfvngttllvdggytar---------
Seq ID No 2  235 lgsdessfmtgselvidggytaq---------
Seq ID No 3  229 llsgrasfitgsyhlvdggyttr---------
Seq ID No 4  241 llsdrasfvagsyhlvdgaytav---------
Seq ID No 5  231 lasddasfivgtslmvdggycaq---------
Seq ID No 6  230 lcsaeasfltggyylvdggytaq---------
Seq ID No 7  227 llsdrasfitgsyhlvdggytal---------
```

Multi-way Aminosäureensequenzvergleich ausgewählter Proteine. Scoring matrix BLOSUM 62. Schattiert dargestellt positionsspezifische identische Aminosäurereste, verglichen mit Referenzsequenz 2bhd_Strex. Eingerahmt sind die von den Erfindern beanspruchten Sequenzmotive.

Überraschenderweise konnte für alle sieben Sequenzen gezeigt werden, dass es sich um NADH-abhängige R-ADHs handelt, die über hinreichende Stabilität, insbesondere gegenüber 2-Propanol, verfügen. Desweiteren konnte für alle sieben Enzyme gezeigt werden, dass sie für enzymatische Reduktionsprozesse mit cosubstratgekoppelter Coenzymregenerierung geeignet sind.

Dies ist ein überraschendes und unerwartetes Ergebnis, insbesondere da die Sequenzen untereinander einen ausgesprochen geringen Grad an Homologie aufweisen (Tabelle 2) und auch bei Sequenzvergleichen (Pubmed/Blast) keine verwandten Sequenzen identifiziert werden konnten, die einen Funktionshinweis in diese Richtung gegeben hätten (Tabelle 1). Auf der anderen Seite konnte wiederum gezeigt werden, dass sich die vorliegenden Enzyme hinreichend in ihren enzymatischen Eigenschaften unterscheiden und sich hinsichtlich ihrer Verfahrenstauglichkeit, insbesondere bei speziellen Targets, komplementieren.
Die Verwendung der genannten Oxidoreduktasen im erfindungsgemäßen Verfahren stellt also keine äquivalente Alternative zum bestehenden Stand der Technik dar, sondern eine Verbreiterung des existierenden Spektrums.

**Tabelle 2**. Paarweiser Aminosäuresequenzvergleich ausgewählter Oxidoreduktasen

| | | SEQ ID NO:1 | SEQ ID NO:2 | SEQ ID NO:3 | SEQ ID NO:4 | SEQID NO:5 | SEO ID NO:6 | SEQ ID NO:7 |
|---|---|---|---|---|---|---|---|---|
| SEQ ID NO | Organismus | Bazillus cereus ATCC 14579 | Methylibium petroleiphilum Pml | Mesorhizobium sp. | Streptomyces coelicolor | Ralstonia | Herpentosiphon aurantiacus | Paracoccus denitrificans |
| SEQ ID NO:1 | Bacillus cereus ATCC 14579 | **100** | **37 / 5** | **41 / 2** | **40 / 6** | **54 / 2** | **41 / 3** | **38 / 2** |
| SEQ ID NO:2 | Methylibium petroleiphilum Pml | **37 / 5** | **100** | **37 / 6** | **33 / 8** | **41 / 5** | **39 / 4** | **43 / 4** |
| SEQ ID NO:3 | Mesorhizobium sp. | **41 / 2** | **37 / 6** | **100** | **56 / 6** | **41 / 2** | **56 / 0** | **64 / 0** |
| SQ ID NO:4 | Streptomyces coelicolor | **40 / 6** | **33 / 8** | **56 / 6** | **100** | **43 / 3** | **53 / 5** | **54 / 5** |
| SQ ID NO:5 | Ralstonia | **54 / 2** | **41 / 5** | **41 / 2** | **43 / 3** | **100** | **45 / 1** | **46 / 4** |
| SQ ID NO:6 | Herpentosiphon aurantiacus | **41 / 3** | **39 / 4** | **56 / 0** | **53 / 5** | **45 / 1** | **100** | **53 / 1** |
| SQ ID NO:7 | Paracoccus denitrificans | **38 / 2** | **43 / 4** | **64 / 0** | **54 / 5** | **46 / 4** | **53 / 1** | **100** |

Der Homologie-Grad in Tabelle 2 ist als Prozent identischer Aminosäurereste und Anzahl von Verschiebungen in einem optimalen paarweisen Alignment angegeben. Das optimale Alignment wird dabei mit Hilfe des BLAST -Algorithmus (Basic Local Alignement Search Tool) ermittelt (Altschul et al. 1990, Proc. Natl. Acd. Sei. USA. 87: 2264-2268). Als Scoring-Matrix zur Berechnung der Sequenzähnlichkeit wird die PAM30-Matrix zugrunde gelegt (Dayhoff; M.O., Schwarz, R.M., Orcutt, B.C. 1978. "A model of evolutionary change in Proteins" in "Atlas of Protein Sequence and structure" 5(3) M. O. Dayhoff (ed) 345-352, National Biomedical Research foundation).

Die Erfindung stellt sich die Aufgabe, ein Verfahren zur enantioselektiven enzymatischen Reduktion von Ketoverbindungen zu den entsprechenden chiralen Hydroxyverbindungen bereitzustellen, welches ein breites Substratspektrum abdeckt und unter prozesstechnischen Bedingungen hohe Umsätze liefert.

Diese Aufgabe wird erfindungsgemäß durch ein Verfahren der eingangs genannten Art gelöst, welches dadurch gekennzeichnet ist, dass zur Reduktion der Ketoverbindungen ein Polypeptid eingesetzt wird, welches eine R-ADH-Signatur H-[P; A]-[I; A; Q; V; L]-[G; K]-R an der Position 205-209 und folgende weitere strukturelle Merkmale in deren Gesamtheit aufweist:

(i) einen N-terminalen Rossmann-Fold GxxxGxG

(ii) ein NAG-Motiv an der Position 87,

(iii) eine katalytische Triade, bestehend aus S 139, Y 152 und K 156,

(iv) einen negativ geladenen Aminosäurerest an Position 37,

(v) zwei C-terminale Motive in der Dimerisierungsdomäne [A; S]-S-F und [V; I]-DG-[G; A]-Y-[T; C; L]-[A; T; S]-[Q; V; R; L; P],

(vi) Val oder Leu an der Position 159,

(vii) Asn an der Position 178, und

(viii) einen Prolinrest an der Position 188.

Gemäß einer bevorzugten Ausführungsform des erfindungsgemäßen Verfahrens wird zur Reduktion der Ketoverbindungen ein Polypeptid eingesetzt,

(a) welches eine der Aminosäuresequenzen SEQ ID NO: 1 bis SEQ ID NO:7 aufweist oder

(b) bei welchem mindestens 70% der Aminosäuren identisch sind mit den Aminosäuren einer der Aminosäuresequenzen SEQ ID NO: 1 bis SEQ ID NO:7 oder

(c) für welches eine Nukleinsäuresequenz aus der Gruppe bestehend aus SEQ ID NO: 8 bis SEQ ID NO: 14 codiert oder

(d) für welches eine Nukleinsäuresequenz codiert, die mit einer der in (c) genannten Nukleinsäureseqenzen unter stringenten Bedingungen hybridisiert.

Unter einer Nukleinsäuresequenz, welche beispielsweise mit der SEQ ID NO: 14 unter stringenden Bedingungen hybridisiert, versteht man ein Polynukleotid, welches mittels der Kolonie-Hybridisierungsmethode, der Plaque-Hybridisierungsmethode, der Southern-Hybridisierungsmethode oder Vergleichbarem unter Verwendung der SEQ ID NO: 14 oder Teilsequenzen von SEQ ID NO: 14 als DNA-Sonde identifiziert werden kann. Zu diesem Zweck wird das an einem Filter immobilisierte Polynukleotid beispielsweise mit der SEQ ID NO: 14 in einer 0,7-1 M NaCl- Lösung bei 65°C hybridisiert. Die Hybridisierung wird, wie z.B. in Protokol 32 von Molecular Cloning, A Laboratory Manual, Second Edition (Cold Spring Harbor Laboratory Press, 1989) oder ähnlichen Publikationen beschrieben, durchgeführt. Anschließend wird der Filter mit 0,1 bis 2-facher SSC-Lösung bei 65°C gewaschen, wobei unter 1-facher SSC-Lösung ein Gemisch, bestehend aus 150 mM NaCl und 15 mM Natriumcitrat, verstanden wird.

Ein Polynukleotid, das mit dem Polynukleotid SEQ ID NO:8, SEQ ID NO:9, SEQ ID NO:10, SEQ ID NO:11, SEQ ID NO:12, SEQ ID NO:13 oder SEQ ID NO:14 unter oben genannten stringenten Bedingungen hybridisiert, sollte zumindest 68% Sequenzidentität mit der Polynukleotidsequenz SEQ ID NO:8, SEQ ID NO:9, SEQ ID NO:10, SEQ ID NO:11, SEQ ID NO:12, SEQ ID NO:13 bzw. SEQ ID NO:14, besser zumindest 80% Identität, noch besser 95% Identität,

aufweisen.

Gemäß einer weiteren bevorzugten Ausführungsform werden Ketoverbindungen der allgemeinen Formel I eingesetzt,

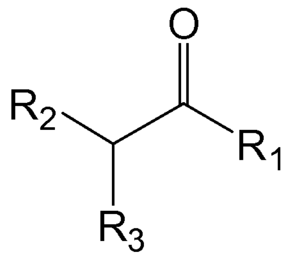

(I)

in der $R_1$, $R_2$ und $R_3$ unabhängig ausgewählt sind aus der Gruppe, bestehend aus

1) -H, mit der Maßgabe, dass $R_1$ nicht H ist,
2) -($C_1$-$C_{20}$)-Alkyl, worin Alkyl geradkettig oder verzweigtkettig ist,
3) -($C_2$-$C_{20}$)-Alkenyl, worin Alkenyl geradkettig oder verzweigtkettig ist und gegebenenfalls bis zu vier Doppelbindungen enthält,
4) -($C_2$-$C_{20}$)-Alkinyl, worin Alkinyl geradkettig oder verzweigtkettig ist und gegebenenfalls bis zu vier Dreifachbindungen enthält,
) -($C_6$-$C_{24}$)-Aryl,
6) -($C_1$-$C_8$)-Alkyl-($C_6$-$C_{14}$)-Aryl,
7) -($C_5$-$C_{14}$)-Heterocyclus,
8) -($C_3$-$C_7$)-Cycloalkyl,

wobei die oben unter 2) bis 8) genannten Reste unsubstituiert sind oder unabhängig voneinander ein-, zwei- oder dreifach durch -OH, Halogen, -$NO_2$, -$NH_2$, -NHP und/oder -M substituiert sind, wobei P für -($C_1$-$C_7$)-Alkyl, -($C_2$-$C_7$)-Alkenyl, -($C_2$-$C_7$)-Alkinyl, -($C_6$-$C_{14}$)-Aryl oder eine Schutzgruppe, ausgewählt aus Benzyloxycarbonyl, Triphenylmethyl und t-Butylcarbonyl, steht, und M für -($C_1$-$C_7$)-Alkyl, -($C_2$-$C_7$)-Alkenyl, -($C_2$-$C_7$)-Alkinyl, -($C_6$-$C_{14}$)-Aryl, oder -($C_1$-$C_8$)-Alkyl-($C_6$-$C_{14}$)-Aryl steht, wobei -($C_6$-$C_{14}$)-Aryl in -($C_1$-$C_8$)-Alkyl-($C_6$-$C_{14}$)-Aryl unsubstituiert oder ein, zwei oder dreifach durch Halogen substituiert ist, und 9) -$CH_2$-X-R, wobei X für O oder S steht und R ausgewählt ist aus -($C_1$-$C_7$)-Alkyl, Phenyl und Benzyl, wobei Phenyl und Benzyl ein-, zwei- oder dreifach durch -($C_1$-$C_7$)-Alkyl, -S($C_1$-$C_3$)-Alkyl, -O($C_1$-$C_3$)-Alkyl, -$NO_2$, -$SCF_3$, Halogen, -C(O)($C_1$-$C_3$)-Alkyl und/oder -CN substituiert sind,
oder $R_1$ mit $R_2$, $R_1$ mit $R_3$ oder $R_2$ mit $R_3$ einen Ring mit 3-6 C-Atomen bildet, welcher unsubstituiert oder unabhängig voneinander ein-, zwei- oder dreifach durch -OH, Halogen, - $NO_2$, -$NH_2$, -NHP und/oder -M substituiert ist, und der übrige Rest aus den oben unter 1) bis 9) genannten Resten ausgewählt ist.

Gemäß einer anderen bevorzugten Ausführungsform werden Diketone der allgemeinen Formel II

(II)

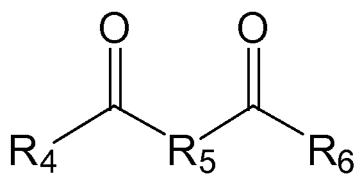

eingesetzt, in der

$R_5$ für $(CH_2)_n$ mit n = 0 - 20, -($C_6$-$C_{14}$)-Aryl oder -($C_5$-$C_{14}$)-Heterocyclus steht,
$R_4$ und $R_6$ unabhängig voneinander für -($C_1$-$C_{20}$)-Alkyl oder eine Estergruppe stehen,
wobei $R_4$, $R_5$ und $R_6$ unsubstituiert sind oder unabhängig voneinander ein-, zwei- oder dreifach durch -($C_1$-$C_4$)-Alkyl, -OH, Halogen, -$NO_2$ und/oder -$NH_2$ substituiert sind.

Beim erfindungsgemäßen Verfahren ist weiters bevorzugt, dass

- der durch die Oxidoreduktase/Dehydrogenase gebildete oxidierte Cofactor NAD kontinuierlich regeneriert wird, und

- zur Cofaktorregenerierung ein sekundärer Alkohol mit der allgemeinen Formel $R_XR_YCHOH$ verwendet wird, wobei $R_X$ und $R_Y$ unabhängig voneinander eine verzweigte oder unverzweigte $C_1$-$C_8$-Alkylgruppe ist und $C_{insgesamt} \geq 3$.

Der Begriff "NAD" bedeutet Nicotinamid-adenin-dinucleotid, der Begriff "NADH" steht für reduziertes Nicotinamid-adenin-dinucleotid.

Die sekundären Alkohole (= Cosubstrate) werden mit Hilfe der eingesetzten Oxidoreduktasen und NAD zu den entsprechenden Ketonen und NADH umgesetzt, wobei es zur Regenerierung des NADH kommt. Der Anteil des Cosubstrates für die Regenerierung beträgt dabei von 5 bis 95 Vol%, bezogen auf das Gesamtvolumen, bevorzugt von 10-70 %, besonders bevorzugt 20-50%.

Besonders bevorzugt wird als sekundärer Alkohol 2-Propanol oder 2-Butanol zur Cofaktorregenerierung eingesetzt. Im Falle von 2-Propanol werden bevorzugt 10-70 % (v/v), besonders bevorzugt 20-50 % (v/v) eingesetzt.

Unter dem Begriff "Aryl" werden aromatische Kohlenstoffreste mit 6 bis 24 Kohlenstoffatomen im Ring / in den (annelierten und durchkonjugierten) Ringen verstanden. -($C_6$-$C_{24}$)-Arylreste sind beispielsweise Phenyl, Naphthyl, zum Beispiel 1-Naphthyl, 2-Naphthyl, Bisphenylyl, zum Beispiel 2-Biphenylyl, 3-Biphenylyl und 4-Biphenylyl, Anthracyl, Fluorenyl, Phenantryl oder Cyclopentanoperhydrophenantryl. Biphenylylreste, Naphthylreste und insbesondere Phenylreste sind bevorzugte Arylreste.
Unter dem Begriff "Halogen" wird ein Element aus der Reihe Fluor, Chlor, Brom oder Jod verstanden.
Unter dem Begriff "-($C_1$-$C_{20}$)-Alkyl" wird ein Kohlenwasserstoffrest verstanden, dessen Kohlenstoffkette geradkettig oder verzweigt ist und 1 bis 20 Kohlenstoffatome enthält, beispielsweise Methyl, Ethyl, Propyl, Isopropyl, Butyl, tertiär-Butyl, Pentyl, Hexyl, Heptyl, Octyl, Nonyl, Decanyl etc.
Unter dem Begriff "-($C_3$-$C_7$)-Cycloalkyl" werden cyclische Kohlenwasserstoffreste verstanden, wie Cyclopropyl, Cylobutyl, Cyclopentyl, Cyclohexyl oder Cycloheptyl.
Der Begriff "-($C_5$-$C_{14}$)-Heterocyclus" steht für einen monocyclischen oder bicyclischen 5-gliedrigen bis 14-gliedrigen heterocyclischen Ring, der teilweise gesättigt oder vollständig gesättigt ist. Beispiele für Heteroatome sind N, O und S. Beispiele für den Begriff "-($C_5$-$C_{14}$)-Heterocyclus" sind Reste, die sich von Pyrrol, Furan, Thiophen, Imidazol, Pyrrazol, Oxazol, Isoxazol, Thiazol, Isothiazol, Tetrazol, 1,2,3,5-Oxathiadiazol-2-oxid, Triazolon, Oxadiazolon, Isoxazolon, Oxadiazolidindion, Triazol, welche z.B. durch F, -CN, -$CF_3$ oder-C(O)-O-($C_1$-$C_4$)-Alkyl substituiert sind, 3-Hydroxypyrro-2,4-dion, 5-Oxo-1,2,4-thiadiazol, Pyridin, Pyrazin, Pyrimidin, Indol, Isoindol, Indazol, Phthalazin, Chinolin, Isochinolin, Chinoxalin, Chinazolin, Cinnolin, Carbolin und benzanellierten, cyclopenta-, cyclohexa- oder cycloheptaanellierten Derivaten dieser Heterocyclen ableiten. Insbesondere bevorzugt sind die Reste 2- oder 3-Pyrrolyl, Phenylpyrrolyl, wie 4- oder 5-Phenyl-2-pyrrolyl, 2-Furyl, 2-Thienyl, 4-Imidazolyl, Methylimidazolyl, zum Beispiel 1-Methyl-2-, -4-oder -5-imidazolyl, 1,3-Thiazol-2-yl, 2-Pyridyl, 3-Pyridyl, 4-Pyridyl, 2-, 3- oder 4-Pyridyl-N-oxid, 2-Pyrazinyl, 2-, 4- oder 5-Pyrimidinyl, 2-, 3- oder 5-Indolyl, substituiertes 2-Indolyl, zum Beispiel 1-Methyl-, 5-Methyl-, 5-Methoxy-, 5-Benzyloxy-, 5-Chlor- oder 4,5-Dimethyl-2-indolyl, 1-Benzyl-2- oder-3-indolyl, 4,5,6,7-Tetrahydro-2-indolyl, Cyclohepta[b]-5-pyrrolyl, 2-, 3- oder 4-Chinolyl, 1-, 3- oder 4-Isochinolyl, 1-Oxo-1,2-dihydro-3-isochinolyl, 2-Chinoxalinyl, 2-Benzofuranyl, 2-Benzothienyl, 2-Benzoxazolyl, Benzothiazolyl oder Dihydropyridinyl, Pyrrolidinyl, zum Beispiel 2- oder 3-(N-Methylpyrrolidinyl), Piperazinyl, Morpholinyl, Thiomorpholinyl, Tetrahydrothienyl oder Benzodioxolanyl.

Bevorzugte Verbindungen der Formel I sind beispielsweise Ethyl-4-chloracetoacetat, Methylacetoacetat, Ethyl-8-chloro-6-oxooctansäure, Ethyl-3-oxovaleriat, 4-Hydroxy-2-butanon, Ethyl-2-oxovaleriat, Ethyl-2-oxo-4-phenylbuttersäure, Ethylpyruvat, Ethylphenylglyoxylat, 1-Phenyl-2-propanon, 2-Chloro-1-(3-chlorphenyl)ethan-1-on, Acetophenon, 2-Octanon, 3-Octanon, 2-Butanon, 1-[3,5-Bis(trifluoromethyl)phenyl]ethan-1-on, 1,4-Dichlor-2-butanon, Acetoxyaceton, Phenacylchlorid, Ethyl-4-bromoacetoacetat, 1,1-Dichloraceton, 1,1,3-Trichloraceton oder 1-Chloraceton.

Besonders bevorzugte Verbindungen sind aromatische oder heteroaromatische Verbindungen der allgemeinen Formel R-CO-CH2-X, wobei X = Halogen und R einen Rest gemäß obiger Definition von Formel I darstellt.

Weitere bevorzugte Verbindungen sind Diketone wie 2,5-Hexandion, 2,4-Pentandion, 2,7-Oktandion, 2,7-Dimethyl-3,6-oktandion oder Diacetyl.

Die Oxidoreduktasen können in dem erfindungsgemäßen Verfahren entweder vollständig gereinigt oder teilweise gereinigt eingesetzt werden oder das Verfahren kann mit Zellen, enthaltend die beschriebenen Oxidoreduktasen, durchgeführt werden. Die eingesetzten Zellen können dabei nativ, permeabilisiert oder lysiert vorliegen. Bevorzugt werden die klonierten Oxidoreduktasen gemäß SEQ ID NO:1, SEQ ID NO:2, SEQ ID NO:3, SEQ ID NO:4, SEQ ID NO:5, SEQ ID NO:6, SEQ ID NO:7 bzw. deren Homologe eingesetzt.

Je kg umzusetzender Ketoverbindung werden 5000 bis 10 Mio U Oxidoreduktase eingesetzt (nach oben offen). Der Enzymeinheit 1 U entspricht dabei die Enzymmenge, die benötigt wird, um 1 $\mu$mol der Ketoverbindung je Minute (min) umzusetzen.

Die Ketoverbindung wird im erfindungsgemäßen Verfahren in einer Menge von 3% bis 50%, bezogen auf das Gesamtvolumen, eingesetzt, bevorzugt von 5% bis 40%, insbesondere von 10%- 30%.

Der in den erfindungsgemäßen Verfahren erreichte TTN (total turn over number = mol reduzierte Ketoverbindung / mol eingesetzter Cofactor) liegt in der Regel im Bereich von $10^2$ bis $10^5$, vorzugsweise beträgt er jedoch $\geq 10^3$.

Zur Regenerierung des Cofactors kann zusätzlich eine Alkoholdehydrogenase zugesetzt werden, bevorzugt wird das Verfahren aber nur mit einer Oxidoreduktase (substratgekoppelte Coenzymregenerierung) durchgeführt.

Der wässrige Anteil des Reaktionsgemisches, in dem die enzymatische Reduktion abläuft, enthält bevorzugt einen Puffer, beispielsweise Kaliumphosphat-, Tris/HCl- oder Triethanolamin-Puffer, mit einem pH-Wert von 5 bis 10, vorzugsweise ein pH-Wert von 6 bis 9. Der Puffer kann zusätzlich noch Ionen zur Stabilisierung oder Aktivierung der Enzyme enthalten, beispielsweise Zinkionen oder Magnesiumionen.

Die Temperatur beträgt während der Durchführung der erfindungsgemäßen Verfahrens zweckmäßig etwa 10°C bis 70°C, bevorzugt 20°C bis 45°C.

In einer weiteren möglichen Ausführungsform des erfindungsgemäßenVerfahrens wird die enzymatische Umsetzung in Anwesenheit von mit Wasser nicht oder nur beschränkt mischbaren organischen Lösungsmitteln durchgeführt, welche nicht an der Cofactorregenerierung beteiligt sind, d.h. keine oxidierbaren Hydroxygruppen enthalten. Dieses Lösungsmittel kann beispielsweise ein symmetrischer oder unsymmetrischer Di($C_1$-$C_6$)alkylether, ein geradkettiges oder verzweigtes Alkan oder Cycloalkan sein. Vorzugsweise werden als zusätzliche organische Lösungsmittel Diethylether, tertiär-Butylmethylether, Diisopropylether, Dibutylether, Ethylacetat, Butylacetat, Heptan, Hexan, Toluol, Dichlormethan, Cyclohexan oder Gemische davon eingesetzt.

Die Konzentration des Cofaktors NAD(H) in der wässrigen Phase beträgt allgemein 0,001 mM bis 1 mM, insbesondere 0,01 mM bis 0,1 mM.

Im erfindungsgemäßen Verfahren kann noch ein Stabilisator der Oxidoreduktase/Dehydrogenase eingesetzt werden. Geeignete Stabilisatoren sind beispielsweise Glycerin, Sorbitol, 1,4 -DL-Dithiothreit (DTT) oder Dimethylsulfoxid (DMSO).

Die enzymatische Reduktion selbst läuft unter milden Bedingungen ab, so dass die erzeugten Alkohole nicht weiterreagieren. Das erfindungsgemäße Verfahren weist eine hohe Standzeit, eine Enantiomerenreinheit von in der Regel mehr als 95% der hergestellten chiralen Alkohole und eine hohe Ausbeute bezogen auf die eingesetzte Menge an Ketoverbindungen auf.

Das erfindungsgemäße Verfahren wird beispielsweise in einem geschlossen Reaktionsgefäß aus Glas oder Metall durchgeführt. Dazu werden die Komponenten einzeln in das Reaktionsgefäß überführt und unter einer Atmosphäre von beispielsweise Stickstoff oder Luft gerührt. Die Reaktionszeit beträgt von 1 Stunde bis 48 Stunden, insbesondere von 2 Stunden bis 24 Stunden.

Anschließend wird das Reaktionsgemisch aufgearbeitet. Dazu wird die wässrige Phase abgetrennt, die organische Phase wird filtriert. Die wässrige Phase kann gegebenenfalls noch einmal extrahiert werden und wie die organische Phase weiter aufgearbeitet werden. Danach wird gegebenenfalls das Lösungsmittel aus der filtrierten organischen Phase verdampft.

Durch die nachstehenden Beispiele wird die Erfindung näher erläutert und die Besonderheit der identifizierten Oxidoreduktasen gegenüber dem Stand der Technik demonstriert.

***Beispiel 1: Klonierung und Expression der Oxidoreduktase SEQ ID NO:5***

A) Anzucht von *Ralstonia eutropha JMP134*

Zellen von *Ralstonia eutropha DSM 4048* wurden in folgendem Medium (pH 7,0) bei 30°C in einem Bakterien-Inkubator kultiviert: 0,5 % Pepton, 0,3 % Fleischextrakt. Am Tag 3 der Kultivierung wurden Zellen mittels Zentrifugation vom Kulturmedium separiert und bei - 80°C gelagert.

B) Amplifikation des für selektive Oxidoreduktase kodierenden Gens

Genomische DNA wurde nach der in "Molecular Cloning" von Manniatis & Sambrook (*supra*) beschriebenen Methode extrahiert. Die resultierende Nukleinsäure diente als Matrize für die Polymerase-Kettenreaktion (PCR) mit spezifischen Primern, die von der in der NCBI Datenbank unter der Nummer 53760931 publizierten Gen-Sequenz abgeleitet wurden. Dabei wurden die Primer für eine nachfolgende Klonierung in einen Expressionsvektor 5'-terminal mit Restriktionsschnittstellen für die Endonukleasen Nde I und Xho I versehen.

Die Amplifizierung wurde in einem PCR-Puffer [10 mM Tris-HCl, (pH 8,0); 50 mM KCl; 10 mM $MgSO_4$; 1 mM dNTP Mix; je 20 pMol Primer und 2,5 U Platinum Pfx DNA-Polymerase (Invitrogen)] mit 500 ng genomischer DNA und folgenden Temperatur-Zyklen durchgeführt:

| | |
|---|---|
| Zyklus 1: | 94°C, 2 min |
| Zyklus 2 x 30: | 94°C, 30 sec |
| | 55°C, 30 sec |

(fortgesetzt)

| | |
|---|---|
| | 68°C, 60 sec |
| Zyklus 3: | 68°C, 7 min |
| | 4°C, ∞ |

Das resultierende PCR-Produkt in einer Größe von etwa 750 bp wurde nach der Reinigung über ein 1 % Agarose-Gel mit Hilfe von Endonukleasen *Nde I* und *Xho I* restringiert und in das mit gleichen Endonukleasen behandelte Rückgrad des pET21a Vectors (Novagen) ligiert. Nach der Transformation von 2 μl des Ligationsansatzes in *E.coli* Top 10 F' Zellen (Invitrogen) wurden Plasmid-DNAs ampicillinresistenter Kolonien mittels einer Restriktionsanalyse mit den Endonukleasen *Nde I* und *Xho I* auf das Vorhandensein eines 750 bp großen Inserts getestet. Plasmid-Präparationen aus den für das Fragment positiven Klonen wurden einer Sequenzanalyse unterzogen und anschließend in *Escherichia coli* BL21 Star (Invitrogen) transformiert.

***Beispiel 2: Expression der rekombinanten Oxidoreduktasen in E.coli***

Die mit dem Expressionskonstrukt transformierten *Escherichia* coli-Stämme BL21 Star (Invitrogen, Karlsruhe, Deutschland), bzw. RB791 (E.coli genetic stock, Yale, USA) wurden in 200 ml LB-Medium (1% Trypton, 0,5% Hefeextrakt, 1% NaCl) mit Ampicillin (50 μg / ml), bzw. Carbenicillin (50 μg / ml) kultiviert, bis eine optische Dichte, gemessen bei 550 nm, von 0,5 erreicht wurde. Die Expression von rekombinantem Protein wurde durch Zugabe von Isopropylthiogalaktosid (IPTG) in einer Konzentration von 0,1 mM induziert. Nach 8 bzw. 16 Stunden Induktion bei 25°C und 220 rpm wurden die Zellen geerntet und bei -20°C eingefroren. Für den Aktivitätstest wurden 10 mg Zellen mit 500 μl 100 mM TEA Puffer pH 7,0 und 500 μl Glasperlen versetzt und 10 min mittels einer Kugelmühle aufgeschlossen. Das erhaltene Lysat wurde dann verdünnt für die entsprechenden Messungen eingesetzt. Der Aktivitätstest setzte sich wie folgt zusammen: 870 μl 100 mM TEA Puffer pH 7,0, 160 μg NAD(P)H, 10 μl verdünntes Zelllysat. Die Reaktion wurde durch Zugabe von 100 μl einer 100 mM Substratlösung zum Reaktionsgemisch gestartet.

Die Oxidoreduktasen der vorliegenden Erfindung ließen sich sehr effizient in *Escherichia coli* exprimieren. Tabelle 3 zeigt die bei der Expression erreichten Aktivitäten der einzelnen Enzyme.

**Tabelle 3**

| | Expressionsvektor | Expressionsstamm | Referenz | Aktivität U/g |
|---|---|---|---|---|
| SEQ ID NO:1 | pET21a | BL21 Star | 2-Octanon | 2650 U/g |
| SEQ ID NO:2 | pET21a | BL21 Star | Methyl-acetoacetat | 1570 U/g |
| SEQ ID NO:3 | pQE70 | BL21 Star | CLAEE | 3130 U/g |
| SEQ ID NO:4 | pET21a | BL21 Star | Ethyl-2-oxo-4-phenylbutanoat | 400 U/g |
| SEQ ID NO:5 | pET21a | BL21 Star | CLAEE | 2890 U/g |
| SEQ ID NO:6 | pQE70 | BL21 Star | Methylacetoacetat | 840 U/g |
| SEQ ID NO:7 | pET21a | BL21 Star | Methylacetoacetat | 3295 U/g |

***Beispiel 3: Charakterisierung der rekombinanten Oxidoreduktasen SEQ ID NO:1 - SEQ ID NO: 7***

*3a: pH-Optimum*

Es wurden die in Tabelle 4 aufgeführten Puffer hergestellt. Die Konzentration der jeweiligen Pufferkomponenten betrug jeweils 50 mM.

Tabelle 4

| PH-Wert | Puffersystem | pH-Wert | Puffersystem |
|---|---|---|---|
| 4 | Na-acetat/Essigsäure | 7,5 | $KH_2PO_4/K_2PO_4$ |
| 4,5 | Na-acetat/Essigsäure | 8 | $KH_2PO_4/K_2PO_4$ |
| 5 | Na-acetat/Essigsäure | 8,5 | $KH_2PO_4/K_2PO_4$ |

(fortgesetzt)

| PH-Wert | Puffersystem | pH-Wert | Puffersystem |
|---|---|---|---|
| 5,5 | $KH_2PO_4/K_2PO_4$ | 9 | Glycin/NaOH |
| 6 | $KH_2PO_4/K_2PO_4$ | 9,5 | Glycin/NaOH |
| 6,5 | $KH_2PO_4/K_2PO_4$ | 10 | Glycin/NaOH |
| 7 | $KH_2PO_4/K_2PO_4$ | 11 | Glycin/NaOH |

Messansatz (30°C)-pH Optimum Reduktion:

| | | |
|---|---|---|
| 870 | μl | der jeweils in Tabelle 3 genannten Puffersysteme |
| 20 | μl | NADH 10 mM |
| 10 | μl | Enzym verdünnt |

Nach etwa 2 bis 3 min Inkubation erfolgte die Zugabe von

100 μl Substratlösung (100mM)

Als Substrat wurde für jede Oxidoreduktase das jeweilige Referenzsubstrat (Tabelle 3) verwendet. Die Reaktion wurde 1 min bei 340 nm verfolgt. Zur Bestimmung des pH-Optimums wurde die enzymatische Reaktion in dem jeweiligen in Tabelle 4 aufgeführten Puffer bestimmt. Zur Bestimmung des pH-Optimums für die Oxidationsreaktion wurde als Cofaktor NAD und als Substrat 2-Propanol oder 2-Oktanol eingesetzt.

In Tabelle 5 sind die Ergebnisse für die Oxidoreduktasen SEQ ID NO:1 - SEQ ID NO:7 zusammengestellt.

Tabelle 5:

| **SEQ ID NO** | **pH-Opt. Red.** | **pH-Opt. Ox.** |
|---|---|---|
| SEQ ID NO:1 | 5,5-6,5 | 9,0-9,5 |
| SEQ ID NO:2 | 5.0-5,5 | 9-10 |
| SEQ ID NO:3 | 5,5 | 10-11 |
| SEQ ID NO:4 | 5,5 | 9-10 |
| SEQ ID NO:5 | 7,0 -7,5 | 9,5-10 |
| SEQ ID NO:6 | 5,5-6,5 | 7,5 |
| SEQ ID NO:7 | 5,5 | 10-11 |

*3b: pH-Stabilität*

Die Stabilität der rekombinanten Oxidoreduktasen wurde durch Lagerung in den in Tabelle 4 genannten Puffersystemen untersucht. Dazu wurden die verschiedenen Puffer (50mM) im Bereich von pH 4 bis 11 angesetzt und die gemäß Beispiel 4 hergestellten Oxidoreduktasen damit verdünnt. Nach 30, 60 und 120 min Inkubation wurden aus dem Ansatz 10 μl entnommen und im Aktivitätstest gemäß Beispiel 3a eingesetzt.

Ausgangswert war dabei der Messwert, den man unmittelbar nach Verdünnung (1:20) des Enzyms in Kaliumphosphatpuffer 50 mM pH = 7,0 erhielt. Dieser Wert entsprach unter den vorgegeben Bedingungen einer Extinktionsänderung von etwa 0,70 /min und wurde als 100%-Wert gesetzt. Alle folgenden Messwerte wurden zu diesem Wert in Relation gesetzt.

In Tabelle 6 sind für die benannten Oxidoreduktasen die pH-Bereiche zusammengestellt, in denen die Enzyme bei 120 min Inkubation nicht weniger als 50% der Ausgangsaktivität aufwiesen.

Tabelle 6:

| SEQ ID NO | pH-Bereich Stabilität |
|---|---|
| SEQ ID NO:1 | 4,5-9,5 |
| SEQ ID NO:2 | 7.0-10 |
| SEQ ID NO:3 | 5,5-11 |
| SEQ ID NO:4 | 5,5-9,5 |
| SEQ ID NO:5 | 7,5-11 |
| SEQ ID NO:6 | 6-11 |
| SEQ ID NO:7 | 5-11 |

*3c: Temperaturoptimum*

Zur Bestimmung der optimalen Testtemperatur wurde die Enzymaktivität für die erfindungsgemäß eingesetzten Oxidoreduktasen im Temperaturbereich von 15°C bis 70°C im Standardmessansatz gemessen.

Die ermittelten Temperaturoptima sind in Tabelle 7 zusammengefasst:

Tabelle 7

| SEQ ID NO | $T_{opt}$ |
|---|---|
| SEQ ID NO:1 | 55°C |
| SEQ ID NO:2 | 65°C |
| SEQ ID NO:3 | n.b |
| SEQ ID NO:4 | n.b |
| SEQ ID NO:5 | 45°C |
| SEQ ID NO:6 | 50°C |
| SEQ ID NO:7 | 45°C |

*3d: Temperaturstabilität*

In analoger Weise, wie unter Beispiel 3c beschrieben, wurde die Temperaturstabilität für den Bereich von 15°C bis 70°C bestimmt. Dazu wurde jeweils eine Verdünnung der erfindungsgemäß eingesetzten Oxidoreduktasen für 60 min und 180 min bei der jeweiligen Temperatur inkubiert und anschließend bei 30°C mit dem obigen Testansatz gemessen. In Tabelle 8 sind für die Oxidoreduktasen die Temperaturbereiche zusammengestellt, in denen die Enzyme bei 120 min Inkubation nicht weniger als 50% der Ausgangsaktivität aufwiesen.

Tabelle 8

| SEQ ID NO | Temperaturbereich |
|---|---|
| SEQ ID NO:1 | 15-45°C |
| SEQ ID NO:2 | 15-35°C |
| SEQ ID NO:3 | n.b |
| SEQ ID NO:4 | n.b |
| SEQ ID NO:5 | 15-35°C |
| SEQ ID NO:6 | 15-35°C |
| SEQ ID NO:7 | 15-45°C |

*3e: Stabilität gegenüber 2-Propanol*

Die Stabilität der zu untersuchenden Oxidoreduktasen gegenüber 2-Propanol wurde untersucht, indem die in Beispiel 2 gewonnenen Lysate (aus rekombinanter Expression) in Puffer (KPP 100 mM, pH=7,0), enthaltend den entsprechenden prozentuellen Anteil an 2-Propanol, verdünnt wurden (ca. 10 Units/ml Puffer). Der Ansatz wurde bei Raumtemperatur unter ständiger Durchmischung (Thermomixer mit 170 rpm) inkubiert. Nach 24 h Inkubation wurden jeweils aus der wässrigen Phase 10 $\mu$l entnommen und für die Bestimmung der Enzymaktivität im Standardtestansatz (Kaliumphosphatpuffer (KPP) 100 mM, pH = 7,0, 0,2 mM NADH, 10 mM Substrat) eingesetzt. Auch hier wurde der Ausgangswert direkt nach Verdünnung im Puffer gleich 100% gesetzt und alle weiteren Werte zu diesem in Relation gesetzt.

Tabelle 9: Enzymaktivität nach 24 h Inkubation mit Gemischen enthaltend 2-Propanol

| SEQ ID NO | Puffer | 10% 2-Propanol | 20% 2-Propanol | 30% 2-Propanol | 40% 2-Propanol |
|---|---|---|---|---|---|
| SEQ ID NO:1 | 100% | 100% | 100% | 100% | 30% |
| SEQ ID NO:2 | 100% | 100% | 100% | 100% | 30% |
| SEQ ID NO:3 | 100% | 80-100% | 50-75% | 25-30% | 0% |
| SEQ ID NO:4 | 100% | 80-100% | 50-75% | 0% | 0% |
| SEQ ID NO:5 | 100% | 100% | 100% | 100% | 0% |
| SEQ ID NO:6 | 100% | 80-100% | 50-75% | 50-75% | 50-75% |
| SEQ ID NO:7 | 100% | 80-100% | 50-75% | 25-30% | 25-30% |

Wie aus Tabelle 9 ersichtlich, weisen alle untersuchten Sequenzen eine erstaunliche Stabilität in 2-Propanol auf, d.h alle Oxidoreduktasen sind in 20% Isopropanol mindestens 24 h stabil, die meisten Enzyme zeigen aber selbst nach 24 h bei 40% (v/v) noch erhebliche Restaktivitäten von bis zu 75%.
Dies zeigt, dass sich die Sequenzen SEQ ID NO:1 - SEQ ID NO:7 insbesondere für die Anwendung im substratgekoppelten Verfahren mit 2-Propanol eignen.

*3f: Vergleich der Substratspektren der Oxidoreduktasen SEQ ID NO: 1 - SEQ ID NO: 7*

Das Substratspektrum der zu charakterisierenden Sequenzen wurde durch Messung der Enzymaktivität für Reduktion und Oxidation mit einer Reihe von Ketonen und Alkoholen bestimmt. Dazu wurde der Standardmessansatz gemäß Beispiel 2 mit unterschiedlichen Substraten verwendet.
Die Aktivität mit Methylacetoacetat wurde für alle Enzyme gleich 100% gesetzt und alle anderen Substrate wurden dazu in Relation gesetzt.

Tabelle 10: Substratspektren / Reduktion

| Substrat | SEQ ID NO:1 | SEQ ID NO:2 | SEQ ID NO:3 | SEQ ID NO:4 | SEQ ID NO:5 | SEQ ID NO:6 | SEQ ID NO:7 |
|---|---|---|---|---|---|---|---|
| 1-Phenyl-2-propanon | 15% | 7% | 70% | 37% | <1% | <1% | 16% |
| Phenacyl-chlorid | 3,4% | 1% | 50% | <1% | <1% | 15% | 10% |
| Acetophenon | 3,5% | 6% | 30% | <1% | <1% | 3,5% | 32% |
| Acetonaphton | 3% | 9% | 30% | 7% | <1% | 25% | 17% |
| Butyrophenon | 3% | 1% | < % | <1% | <1% | <1% | <1% |
| 2-Octanon | 92% | 2% | 33% | 70% | 26% | 23% | 49% |
| 3-Octanon | 22% | 2% | 15% | 5% | 13% | 2% | 15% |
| 2-Butanon | 8,5% | 4,5% | 30% | 5% | <1% | 7% | 6% |
| Ethyl-2-oxovaleriat | 3% | 60% | 70% | 17% | <1% | 15% | <1% |
| Ethyl-2-oxo-4-phenylbuttersäure | 3% | 50% | 118% | 35% | 45% | 18% | 10% |

(fortgesetzt)

| Substrat | SEQ ID NO:1 | SEQ ID NO:2 | SEQ ID NO:3 | SEQ ID NO:4 | SEQ ID NO:5 | SEQ ID NO:6 | SEQ ID NO:7 |
|---|---|---|---|---|---|---|---|
| Ethylpyruvat | 140% | 70% | 100% | 64% | 13% | 116% | 160% |
| Ethylphenylglyoxylat | 4,8% | 53% | 7,5% | 3% | <1% | <1% | <1% |
| Ethyl-4-Chloroacetoacetat | 12,5% | 20% | 21% | 100% | 50% | 10% | <1% |
| **Methylacetoacetat** | **100%** | **100%** | **100%** | **100%** | **100%** | **100%** | **100%** |
| Ethyl-3-oxovaleriat | 3,5% | 30% | 76% | 11% | <1% | 10% | 12% |
| Aceton | 4,5% | 16% | 38% | 5% | <1% | 3,5% | 8% |

Für ausgewählte Substrate wurden die Enantiomerenüberschüsse bestimmt. Dazu wurde folgender Reaktionsansatz verwendet:

- 160 µl Puffer
- 100 µl NAD (0,4 mg/ml)
- 20-50 µl 2-Propanol
- 50 µl Enzymlösung
- 2 mg Substrat
- Reaktionsbedingungen: 28°C, 24h

Nach Ende der Inkubationszeit wurden die Proben mit Lösungsmitteln je nach Substrat extrahiert und zentrifugiert. Ein Aliquot wurde entnommen, ggf. das Extraktionsmittel komplett entfernt, die Probe anschließend in einem geeigneten Lösungsmittel gelöst und der Enantiomerenüberschuss mittels GC bestimmt.

Tabelle 11: Enantioselektivität für ausgewählte Substrate

| Substrat | SEQ ID NO:1 | SEQ ID NO:2 | SEQ ID NO:3 | SEQ ID NO:4 | SEQ ID NO:5 | SEQ ID NO:6 | SEQ ID NO:7 |
|---|---|---|---|---|---|---|---|
| Phenacyl-chlorid | ≥99,9% S | ≥99,9% S | ≥99,9% S | 97,5% S | 60-80% S | ≥99,9% S | ≥99,9% S |
| Acetophenon | 99,0% R | 98,5% R | 99,0% R | | | 99,0% R | 98,5% R |
| 2-Pentanon | ≥99,9% R | 98% R | 98% R | 95% R | 99,0% R | 97% R | 99,5% R |
| 2-Octanon | ≥99,9% R | ≥99,9% R | ≥99,9% R | ≥99,9% R | ≥99,9% R | ≥99,9% R | ≥99,9% R |
| 2-Butanon | ≥96,0% R | n.b | n.b | n.b | n.b | rac | 50% R |
| Ethylpyruvat | 98,0% R | ≥99,9% R | ≥99,9% R | ≥99,9% R | ≥99,9% R | ≥99,9% R | ≥99,9% R |
| Ethyl-4-Chloroacetoacetat | 99,0% S | ≥99,9% S | ≥99,9% S | ≥99,9% S | ≥99,9% S | ≥99,9% S | ≥99,9% S |
| Methylacetoacetat | ≥99,9% R | ≥99,9% R | ≥99,9% R | 98,5% R | ≥99,9% R | ≥99,9% R | ≥99,9% R |
| "rac": unselektive Reduktion, d.h. beide Enantiomere entstehen in ungefähr gleichem Verhältnis<br>n.b = nicht bestimmt, keine Umsetzung | | | | | | | |

Der Enantiomerenüberschuss wird wie folgt berechnet:

$$ee(\%) = ((R\text{-Alkohol} - S\text{-Alkohol})/(R\text{-Alkohol} + S\text{-Alkohol})) \times 100.$$

Das Potential der beschriebenen Oxidoreduktasen in Richtung Oxidation und somit Regenerationstauglichkeit wurde verglichen, indem folgender Aktivitätstest verwendet wurde: 870 µl 100 mM TEA Puffer, pH 8,0, 160 µg NAD, 10 µl verdünntes Zelllysat. Die Reaktion wurde durch Zugabe von 100 µl einer 100 mM Substratlösung zum Reaktionsgemisch gestartet.

In diesem Fall wurde das Substrat mit der höchsten Aktivität gleich 100% gesetzt.

Tabelle 12: Substratspektren / Oxidation

| Substrat | SEQ ID NO:1 | SEQ ID NO:2 | SEQ ID NO:3 | SEQ ID NO:4 | SEQ ID NO:5 | SEQ ID NO:6* | SEQ ID NO:7 |
|---|---|---|---|---|---|---|---|
| S-2-Oktanol | 0% | 0% | 0% | 0% | 0% | 0% | 0% |
| R-2-Oktanol | **100%** | 31% | **100%** | **100%** | **100%** | **100%** | **100%** |
| S-2-Butanol | 1% | 20% | 30% | 2% | 2,5% | <2% | 0% |
| R-2-Butanol | 3% | **100%** | 58% | 18% | 14% | 60% | 50% |
| S-Phenyl-2-propanol | 0% | 0% | 0% | 0% | 0% | 0% | 0% |
| R-Phenyl-2-propanol | <1% | 20% | 50% | 2% | 25% | 0% | 50% |
| 2-Methyl-4-pentanol | <1% | 20% | 10% | 1% | 2,5% | 0% | 0% |
| 2-Propanol | 1,6% | 20 % | **100%** | 7% | 7% | 60% | 20% |
| Cyclohexanol | 0% | 20% | 0% | 0% | 0% | 0% | 0% |
| *Oxidation kaum nachweisbar | | | | | | | |

Wie aus den Tabellen 10-12 ersichtlich ist, handelt es sich bei allen sieben Sequenzen deutlich um R-spezifische Oxidoreduktasen. Die untersuchten Enzyme unterscheiden sich aber deutlich im Spektrum präferierter Substrate sowie im Oxidationsverhalten. Unterschiede treten dabei hinsichtlich der Präferenz kurzkettiger, eher niedermolekularer Substrate gegenüber langkettigen Carbonylverbindung auf, ebenso werden unterschiedliche aromatische Systeme oder auch verschiedene Substituenten (wie Halogen) unterschiedlich gut toleriert.

Obwohl man also eine Auswahl von Enzymen hat, die zwar prinzipiell ähnliche Reaktionen katalysieren, stellt man für einzelne Substrate fest, dass man darunter sowohl solche Enzyme findet, die die Targetreaktion extrem effizient katalysieren, als auch solche, die die Targetreaktion überhaupt nicht katalysieren.

Desweiteren beobachtet man auch Unterschiede in der Enantioselektivität, die zwar auf den ersten Blick geringfügig erscheinen, aber bei einer heute für pharmazeutische Anwendungen üblichen Spezifikation von ee>99,0-99,9 % durchaus entscheidend für die Anwendbarkeit einzelner Enzyme für spezifische Reduktionsprozesse sein können.

Auch die oxidativen Eigenschaften und somit die Anwendbarkeit in verschiedenen Prozessen mit substratgekoppelter Coenzymregenerierung variieren zwischen den untersuchten Enzymen sowie auch zum Stand der Technik. Die Verwendung der benannten Oxidoreduktasen im erfindungsgemäßen Verfahren stellt also keine äquivalente Alternative zum bestehenden Stand der Technik dar, sondern eine Verbreiterung des existierenden Spektrums.

***Beispiel 4: Vergleich der Oxidoreduktasen SEQ ID NO:1- SEQ ID NO: 7 in speziellen Verfahren***

Anhand der unten aufgeführten Beispiele soll nun für einige spezielle Prozesse gezeigt werden, dass die Enzyme SEQ ID NO:1 bis 7 spezielle Lösungen zu konkreten Fragestellungen liefern können, die auch deutlich über den vorhandenen Stand der Technik hinausgehen.

*4. a Reduktion von 3,6-Oktandion zu R,R-3,6-Oktandiol*

Zum Screening nach geeigneten Enzymen zur Entwicklung eines Prozesses mit substratgekoppelter Coenzymregenerierung zur Reduktion von 3,6-Oktandion zu R,R-3,6-Oktandiol wurden sämtliche Enzyme in folgendem Reaktionsansatz untersucht:

450 µl Puffer, pH =7,0

0,05 mg NAD
50 μl 2-Propanol
10 mg 3,6-Oktandion

Nach 24 h Inkubation bei 25°C wurden die Proben mittels Dichlormethan extrahiert und durch GC analysiert.

In Tabelle 13 sind die erhaltenen Umsätze und Reaktionsprodukte angegeben.

Tabelle 13

| SEQ ID NO | 3,6-Octandion | einfach reduziertes Produkt(S) | einfach reduziertes Produkt (R) | S,S-3,6-Oktandiol | R,R-3,6-Oktandiol |
|---|---|---|---|---|---|
| SEQ ID NO: 1 | 95% | 0% | 5% | 0% | 0% |
| SEQ ID NO:2 | 3% | 0% | 17% | 0% | 80% |
| SEQ ID NO:3 | 10% | 0% | 45% | 0% | 45% |
| SEQ ID NO:4 | 76% | 0% | 24% | 0% | 0% |
| SEQ ID NO:5 | 95% | 0% | 5% | 0% | 0% |
| SEQ ID NO:6 | 24% | 0% | 59% | 0% | 17% |
| SEQ ID NO:7 | 43% | 0 | 47% | 0 | 10% |
| WO 2007/012428 *Pichia farinosa* | 85% | 0% | 15% | 0% | 0% |
| WO 2007/012428 *Candida nemodendra* | 95% | 0% | 5% | 0% | 0% |
| EP 1179 595 A1 *Pichia finlahdica* | 3% | 0% | 17% | 0% | 80% |
| WO 2004/027055 *Devosia* | 40% | 0% | 50% | 0% | 10% |

Wie aus Tabelle 13 ersichtlich, findet man unter den zur Verfügung stehenden Enzymen solche, die unter den genannten Reaktionsbedingungen sehr gut in der Lage sind, 3,6-Oktandion zum gewünschten R,R-Oktandiol zu reduzieren (SEQ ID NO:2, SEQ ID NO:3 und *P.finlandica*), solche, die das Substrat so gut wie gar nicht akzeptieren (SEQ ID NO:1, SEQ ID NO:5 und C. *nemodendra*) sowie auch solche, die man bevorzugt verwenden müsste, wenn man beabsichtigt, die einfach reduzierte Verbindung herzustellen (SEQ ID NO:4).

*4.b Reduktion von 2,7-Dimethyl-3,6-oktandion zu (S,S)-2,7-Dimethyloktan-3,6-diol*

Zum Screening nach geeigneten Enzymen für einen Prozess mit substratgekoppelter Coenzymregenerierung zur Reduktion von 2,7-Dimethyl-3,6-oktandion zu (S,S)-2,7-Dimethyloktan-3,6-diol wurden die Enzyme in folgendem Reaktionsansatz untersucht:

450 μl Puffer, pH =7,0
0,05 mg NAD
50 μl 2-Propanol
10 mg 2,7-Dimethyl-3,6 oktandion

Nach 24 h Inkubation bei 25°C wurden die Proben mittels Dichlormethan extrahiert und durch GC analysiert.

In Tabelle 14 sind die erhaltenen Umsätze und Reaktionsprodukte angeführt.

Tabelle 14

| SEQ ID NO | 2,7-Dimethyl-3,6-oktandion | Meso | (S,S)-2,7-Dimethyloktan-3,6-diol | (R,R)-2,7-Dimethyl-3,6-oktandiol |
|---|---|---|---|---|
| SEQ ID NO:1 | 100% | 0% | 0% | 0% |

(fortgesetzt)

| SEQ ID NO | 2,7-Dimethyl-3,6-oktandion | Meso | (S,S)-2,7-Dimethyloktan-3,6-diol | (R,R)-2,7-Dimethyl-3,6-oktandiol |
|---|---|---|---|---|
| **SEQ ID NO:2** | **23%** | **0** | **77%** | **0%** |
| SEQ ID NO:3 | 100% | 0% | 0% | 0% |
| SEQ ID NO:4 | 100% | 0% | 0% | 0% |
| SEQ ID NO:5 | 100% | 0% | 0% | 0% |
| SEQ ID NO:6 | 100% | 0% | 0% | 0% |
| SEQ ID NO:7 | 100% | 0% | 0% | 0% |
| WO 2007/012428 *Pichia farinosa* | 100% | 0% | 0% | 0% |
| WO 2007/012428 *Candida nemodendra* | 100% | 0% | 0% | 0% |
| EP 1179 595 A1 *Pichia finlandica* | 100% | 0% | 0% | 0% |
| WO 2004/027055 *Devosia* | 100% | 0% | 0% | 0% |

Wie aus Tabelle 14 ersichtlich ist, liefert für diese spezifische Fragestellung SEQ ID NO:2 eine Lösung, hingegen ist kein Enzym aus dem Stand der Technik in der Lage, die gewünschte Reaktion zu katalysieren.

*4.c Reduktion von 2,4-Pentandion zu R,R-2,4-Pentandiol*

Zum Screening nach geeigneten Enzymen für einen Prozess mit substratgekoppelter Coenzymregenerierung zur Reduktion von 2,4-Pentandion zu R,R-2,4-Pentandiol wurden die Enzyme in folgendem Reaktionsansatz untersucht:

450 µl Puffer, pH =7,0
0,05 mg NAD
50 µl 2-Propanol
10 mg 2,4 -Pentandion

Nach 24 h Inkubation bei 25°C wurden die Proben mittels Dichlormethan extrahiert und durch GC analysiert.
In Tabelle 15 sind die erhaltenen Umsätze und Reaktionsprodukte angeführt.

Tabelle 15

| SEQ ID NO | 2,4-Pentandion | einfach reduzierte Verbindung (R) | (S,S)-2,4-Pentandiol | (R,R)-2,4-Pentandiol |
|---|---|---|---|---|
| SEQ ID NO: 1 | 0% | 70% | 0% | 30% |
| **SEQ ID NO:2** | **0%** | **19%** | **0%** | **81%** |
| SEQ ID NO:3 | 2% | 95% | 0% | 3% |
| SEQ ID NO:4 | 6% | 93% | 0% | 1% |
| SEQ ID NO:5 | 60% | 40% | 0% | 0% |
| SEQ ID NO:6 | 100% | 0% | 0% | 0% |
| SEQ ID NO:7 | 7% | 91% | 0% | 2% |
| WO 2007/012428 *Pichia farinosa* | 100% | 0% | 0% | 0% |

(fortgesetzt)

| SEQ ID NO | 2,4-Pentandion | einfach reduzierte Verbindung (R) | (S,S)-2,4-Pentandiol | (R,R)-2,4-Pentandiol |
|---|---|---|---|---|
| WO 2007/012428 *Candida nemodendra* | 55% | 45% | 0% | 0% |
| EP 1179 595 A1 *Pichia finlandica* | 30% | 70% | 0% | 0% |
| WO 2004/027055 *Devosia* | 43% | 57% | 0% | 0% |

Wie aus Tabelle 15 ersichtlich, ist die Umsetzung von 2,4-Pentandion zu R,R-2,4-Pentandiol mit den Oxidoreduktasen SEQ ID NO:1 und SEQ ID NO:2 möglich. Die meisten Enzyme setzen dieses Substrat nur bis zur monoreduzierten Verbindung um. Auch der Stand der Technik bietet hier keine Lösung.

Zur effizienten Gewinnung der monoreduzierten Verbindung wiederum empfehlen sich die Oxidoreduktasen SEQ ID NO:3, SEQ ID NO:4 und SEQ ID NO:7 noch vor dem Stand der Technik.

*4.e Reduktion von 1,1,1-Trifluoraceton zu R-1,1,1-Trifluoropropanol*

Zum Screening nach geeigneten Enzymen für einen Prozess mit substratgekoppelter Coenzymregenerierung zur Reduktion von 1,1,1-Trifluoraceton zu R-1,1,1 Trifluoropropanol wurden die Enzyme in untenstehendem Reaktionsansatz untersucht.

Der Reaktionsansatz trägt dabei der besonderen Erfordernis an die Aufarbeitung und Umsetzung dieses hochflüchtigen Substrates Rechnung. Hier ist die Regeneration mit Isopropanol nicht möglich,vielmehr ist hier die Verwendung von Methylpentanol bevorzugt, um zum einen ein Entweichen des flüchtigen Substrates (Sp = 22°C) während der Reaktion zu vermeiden und zum anderen die Aufarbeitung des Reaktionsproduktes R-1,1,1 Trifluoropropanol (Sp =80°C) zu ermöglichen. (Die Abtrennung von 2-Propanol wäre aufgrund identischer Siedpunkte nicht möglich.)

450 µl Puffer, pH =7,0
0,05 mg NAD
500 µl 2-Methyl-4-pentanol
20 µl 1,1,1-Trifluoraceton

Nach 24 h Inkubation bei 25°C wurden die Proben durch GC analysiert.

In Tabelle 16 sind die erhaltenen Umsätze und Reaktionsprodukte angeführt.

Tabelle 16

| SEQ ID NO | 1,1,1-Trifluoraceton | R-1,1,1-Trifluoropropanol | S-1,1,1-Trifluoropropanol |
|---|---|---|---|
| **SEQ ID NO:1** | **0%** | **99%** | **1%** |
| SEQ ID NO:2 | 20% | 60% | 40% |
| SEQ ID NO:3 | 2% | 70% | 30% |
| SEQ ID NO:4 | 15% | 80% | 20% |
| SEQ ID NO:5 | 50% | 85% | 15% |
| SEQ ID NO:6 | n.b | n.b | n.b |
| SEQ ID NO:7 | n.b | n.b | n.b |
| WO 2007/012428 *Pichia farinosa* | 0% | 97% | 3% |
| WO 2007/012428 *Candida nemodendra* | 50% | 99% | 1% |
| EP 1179 595 A1 *Pichia finlandica* | 50% | 99% | 1% |
| WO 2004/027055 *Devosia* | n.b | n.b | n.b |

Wie in Tabelle 16 ersichtlich, bietet hier die Oxidoreduktase SEQ ID NO:1 den besten Kompromiss zwischen Umsatz und Selektivität. Erstaunlicherweise sind die meisten Enzyme überhaupt nicht in der Lage, dieses Substrat mit befriedigender Enantioselektivität zu reduzieren, wodurch nochmals die Diversität der beschriebenen Enzyme bei prinzipiell gleicher Funktionalität unterstrichen wird.

*4.f Reduktion von 2-Chloro-1-(3-hydroxyphenyl)ethan-1-on zu (1S)-2-Chloro-1-(3-hydroxyphenyl)ethan-1-ol*

Zum Screening nach geeigneten Enzymen für einen Prozess mit substratgekoppelter Coenzymregenerierung zur Reduktion von 2-Chloro-1-(3-hydroxyphenyl)ethan-1-on zu (1S)-2-Chloro-1-(3-hydroxyphenyl)ethan-1-ol wurden die Enzyme in folgendem Reaktionsansatz untersucht:

400 μl Puffer, pH =7,0
0,02 mg NAD
100 μl 2-Propanol
25 mg 2-Chloro-1-(3-hydroxyphenyl)ethan-1-on

Nach 24 h Inkubation bei 25°C wurden die Proben extrahiert und durch GC analysiert.
In Tabelle 17 sind die erhaltenen Umsätze und Reaktionsprodukte angeführt.

Tabelle 17

| SEQ ID NO | 2-Chloro-1-(3-hydroxyphenyl)ethan-1-on | (1R)-2-Chloro-1-(3-hydroxyphenyl)ethan-1-ol | (1S)-2-Chloro-1-(3-hydroxyphenyl)ethan-1-ol |
|---|---|---|---|
| **SEQ ID NO:1** | **100%** | **n.b** | **n.b** |
| SEQ ID NO:2 | 55% | 0% | 45% |
| SEQ ID NO:3 | 40% | 0% | 60% |
| SEQ ID NO:4 | 99% | n.b | n.b |
| SEQ ID NO:5 | **100%** | **n.b** | **n.b** |
| SEQ ID NO:6 | 25% | 0% | 75% |
| SEQ ID NO:7 | 83% | 0% | 17% |
| WO 2007/012428 *Pichia farinosa* | **100%** | **n.b** | **n.b** |
| WO 2007/012428 *Candida nemodendra* | **100%** | **n.b** | **n.b** |
| EP 1179 595 A1 *Pichia finlandica* | 98% | n.b | n.b |
| WO 2004/027055 *Devosia* | **100%** | **n.b** | **n.b** |

Wie aus Tabelle 17 ersichtlich, ist die Umsetzung von 2-Chloro-1-(3-hydroxyphenyl)ethan-1-on zu (1S)-2-Chloro-1-(3-hydroxyphenyl)ethan-1-ol prinzipiell mit den Oxidoreduktasen SEQ ID NO:2, SEQ ID NO:3, SEQ ID NO:6 und SEQ ID NO:7 möglich, wobei diese Enzyme das Substrat absolut selektiv umsetzen. Kein Enzym aus dem Stand der Technik ist in der Lage, dieses spezielle Substrat zu reduzieren.

*4.g Reduktion von Ethyl-4-chloroacetoacetat zu (S)-Ethyl-4-chloro-3-hydroxybutyrat*

Im folgenden werden die zu untersuchenden Enzyme SEQ ID NO:1-7 im industriellen Prozess zur Reduktion

von Ethyl-4-chloroacetoacetat zu (S)-Ethyl-4-chloro-3-hydroxybutyrat untersucht und mit dem Stand der Technik verglichen.

Dazu wurde das Substrat Ethyl-4-chloroacetoacetat entsprechend dem Stand der Technik (WO 2006/087235) in hoher Konzentration (200 g/l) eingesetzt und die Effizienz der Enzyme im Prozess mit substratgekoppelter Regeneration mit 2-Propanol verglichen.

600 µl Puffer, pH =8,0
0,2 mg NAD
200 µl 2-Propanol
200 µl Ethyl-4-chloroacetoacetat
240µl Enzym rekombinant aus *E.coli* (20-60 Units)

Nach 24 h Inkubation bei 25°C wurden die Proben extrahiert und durch GC analysiert.

In Tabelle 18 sind die erhaltenen Umsätze und Reaktionsprodukte angeführt.

Tabelle 18

| SEQ ID NO | Ethyl-4-chloroacetoacetat | (S)-Ethyl-4-chloro-3-hydroxybutyrat | (R)-Ethyl-4-chloro-3-hydroxybutyrat |
|---|---|---|---|
| SEQ ID NO:1 | 90% | 10% | 0% |
| **SEQ ID NO:2** | **0%** | **100%** | **0%** |
| **SEQ ID NO:3** | **0%** | **100%** | **0%** |
| SEQ ID NO:4 | 70% | 30% | 0% |
| SEQ ID NO:5 | 85% | 15% | 0% |
| SEQ ID NO:6 | 60% | 40% | 0% |
| SEQ ID NO:7 | 75% | 25% | 0% |
| WO 2007/012428 *Pichia farinosa* | 100% | n.b | n.b |
| WO 2007/012428 *Candida nemodendra* | 100% | n.b | n.b |
| EP 1179 595 A1 *Pichia finlandica* | 80% | 20% | 0% |
| WO 2004/027055 *Devosia* | 75% | 25% | 0% |

Wie aus Tabelle 18 ersichtlich, bieten insbesondere die Enzyme SEQ ID NO:2 und SEQ ID NO:3 für die Reduktion von Ethyl-4-chloroacetoacetat zu (S)-Ethyl-4-chloro-3-hydroxybutyrat interessante, NADH-abhängige Alternativen zu den bestehenden, meist NADPH-abhängigen Verfahren. Die Enzyme aus dem Stand der Technik sind hingegen ungeeignet, da sie unter Verfahrensbedingungen nur ungenügende bzw. gar keine Umsätze zeigen.

## Patentansprüche

1. Verfahren zur stereoselektiven, insbesondere enantioselektiven enzymatischen Reduktion von Ketoverbindungen zu den entsprechenden chiralen Hydroxyverbindungen, wobei die Ketoverbindungen mit einer bakteriellen enantioselektiven, NADH-spezifischen Oxidoreduktase reduziert werden, **dadurch gekennzeichnet, dass** zur Reduktion der Ketoverbindungen ein Polypeptid eingesetzt wird, welches eine R-ADH-Signatur H-[P; A]-[I; A; Q; V; L]-[G; K]-R an der Position 205-209 und folgende weitere strukturelle Merkmale in deren Gesamtheit aufweist:

   (i) einen N-terminalen Rossmann-Fold GxxxGxG,
   (ii) ein NAG-Motiv an der Position 87,
   (iii) eine katalytische Triade, bestehend aus S 139, Y 152 und K 156,
   (iv) einen negativ geladenen Aminosäurerest an Position 37,
   (v) zwei C-terminale Motive in der Dimerisierungsdomäne [A; S]-S-F und [V; I]-DG-[G; A]-Y-[T; C; L]-[A; T; S]-[Q; V; R; L; P],
   (vi) Val oder Leu an der Position 159,
   (vii) Asn an der Position 178, und
   (viii) einen Prolinrest an der Position 188,

   wobei die Position der strukturellen Merkmale bestimmt ist durch Multi-way Aminosäurenvergleich mit Scoring-Matrix BLOSUM 62 mit der Referenzsequenz 2bhd_Strex mit der Aminosäuresequenz:

   MHDLTGKNVIITGGARGLGAEAARQAVAAGAHVLITDVLDDDGENAARELGDRARFLHHD
   VTSEEDWSRAADFAVTEFGALHGLVNNAGISTGTPLESESVDHFRKVLDVNLTGVFIGMK
   TVVPALKEAGGGSIVNISSAAGLMGLALTAGYGASKWGVRGLTKIGAVEWGTARVRVNSV
   HPGMTYTPMTAAVGIERGEGKYPNTPMGRVGEADEIAGAVVFLLSDAASYVTGAELAVDG
   GWTTGPTVAYVMGQ

   und den Aminosäuresequenzen SEQ ID NO:1 bis SEQ ID NO:7.

2. Verfahren nach Anspruch 1, **dadurch gekennzeichnet, dass** zur Reduktion der Ketoverbindungen eine funktionelle Oxidoreduktase eingesetzt wird,

   (a) welche eine der Aminosäuresequenzen SEQ ID NO:1 bis SEQ ID NO:7 aufweist oder
   (b) bei welcher mindestens 70% der Aminosäuren identisch sind mit den Aminosäuren einer der Aminosäuresequenzen SEQ ID NO:1 bis SEQ ID NO:7 oder
   (c) für welche eine Nukleinsäuresequenz aus der Gruppe bestehend aus SEQ ID NO:8 bis SEQ ID NO:14 codiert oder
   (d) für welche eine Nukleinsäuresequenz codiert, die mit einer der in (c) genannten Nukleinsäureseqenzen unter stringenten Bedingungen hybridisiert.

3. Verfahren nach Anspruch 1 oder 2, **dadurch gekennzeichnet, dass** Ketoverbindungen der allgemeinen Formel I eingesetzt werden,

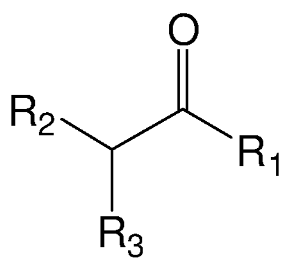

(I)

   in der $R_1$, $R_2$ und $R_3$ unabhängig ausgewählt sind aus der Gruppe, bestehend aus

1) -H, mit der Maßgabe, dass $R_1$ nicht H ist,
2) -($C_1$-$C_{20}$)-Alkyl, worin Alkyl geradkettig oder verzweigtkettig ist,
3) -($C_2$-$C_{20}$)-Alkenyl, worin Alkenyl geradkettig oder verzweigtkettig ist und gegebenenfalls bis zu vier Doppelbindungen enthält,
4) -($C_2$-$C_{20}$)-Alkinyl, worin Alkinyl geradkettig oder verzweigtkettig ist und gegebenenfalls bis zu vier Dreifachbindungen enthält,
5) -($C_6$-$C_{24}$)-Aryl,
6) -($C_1$-$C_8$)-Alkyl-($C_6$-$C_{14}$)-Aryl,
7) -($C_5$-$C_{14}$)-Heterocyclus,
8) -($C_3$-$C_7$)-Cycloalkyl,

wobei die oben unter 2) bis 8) genannten Reste unsubstituiert sind oder unabhängig voneinander ein-, zwei- oder dreifach durch -OH, Halogen, -$NO_2$, -$NH_2$, -NHP und/oder -M substituiert sind, wobei P für -($C_1$-$C_7$)-Alkyl, -($C_2$-$C_7$)-Alkenyl, -($C_2$-$C_7$)-Alkinyl, -($C_6$-$C_{14}$)-Aryl oder eine Schutzgruppe, ausgewählt aus Benzyloxycarbonyl, Triphenylmethyl und t-Butylcarbonyl, steht, und M für -($C_1$-$C_7$)-Alkyl, -($C_2$-$C_7$)-Alkenyl, -($C_2$-$C_7$)-Alkinyl, -($C_6$-$C_{14}$)-Aryl, oder -($C_1$-$C_8$)-Alkyl-($C_6$-$C_{14}$)-Aryl steht, wobei -($C_6$-$C_{14}$)-Aryl in -($C_1$-$C_8$)-Alkyl-($C_6$-$C_{14}$)-Aryl unsubstituiert oder ein, zwei oder dreifach durch Halogen substituiert ist, und

9) -$CH_2$-X-R, wobei X für O oder S steht und R ausgewählt ist aus -($C_1$-$C_7$)-Alkyl, Phenyl und Benzyl, wobei Phenyl und Benzyl ein-, zwei- oder dreifach durch -($C_1$-$C_7$)-Alkyl, -S($C_1$-$C_3$)-Alkyl, -O($C_1$-$C_3$)-Alkyl, -$NO_2$, -$SCF_3$, Halogen, -C(O)($C_1$-$C_3$)-Alkyl und/oder -CN substituiert sind,

oder $R_1$ mit $R_2$, $R_1$ mit $R_3$ oder $R_2$ mit $R_3$ einen Ring mit 3-6 C-Atomen bildet, welcher unsubstituiert oder unabhängig voneinander ein-, zwei- oder dreifach durch -OH, Halogen, - $NO_2$, -$NH_2$, -NHP und/oder -M substituiert ist, und der übrige Rest aus den oben unter 1) bis 9) genannten Resten ausgewählt ist.

**4.** Verfahren nach Anspruch 1 oder 2, **dadurch gekennzeichnet, dass** als Ketoverbindungen Diketone der allgemeinen Formel II

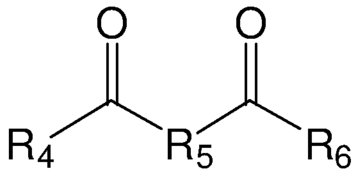

(II)

eingesetzt werden, in der

$R_5$ für $(CH_2)_n$ mit n = 0 - 20, -($C_6$-$C_{14}$)-Aryl oder -($C_5$-$C_{14}$)-Heterocyclus steht,
$R_4$ und $R_6$ unabhängig voneinander für -($C_1$-$C_{20}$)-Alkyl oder eine Estergruppe stehen,
wobei $R_4$, $R_5$ und $R_6$ unsubstituiert sind oder unabhängig voneinander ein-, zwei- oder dreifach durch -($C_1$-$C_4$)-Alkyl, -OH, Halogen, -$NO_2$ und/oder -$NH_2$ substituiert sind.

**5.** Verfahren nach einem der Ansprüche 1 bis 4, **dadurch gekennzeichnet, dass**

- der durch die Oxidoreduktase/Dehydrogenase gebildete oxidierte Cofactor NAD kontinuierlich regeneriert wird, und
- zur Cofaktorregenerierung ein sekundärer Alkohol mit der allgemeinen Formel $R_XR_YCHOH$ verwendet wird, wobei $R_X$ und $R_Y$ unabhängig voneinander eine verzweigte oder unverzweigte $C_1$-$C_8$-Alkylgruppe ist und $C_{insgesamt} \geq 3$.

**6.** Verfahren nach Anspruch 5, **dadurch gekennzeichnet, dass** als Cosubstrat bzw. sekundärer Alkohol ein Alkohol aus der Gruppe bestehend aus 2-Propanol, 2-Butanol, 2-Pentanol, 4-Methyl-2-pentanol, 2-Heptanol und 2-Octanol, vorzugsweise 2- Propanol, eingesetzt wird.

**7.** Verfahren nach einem der Ansprüche 1 bis 6, **dadurch gekennzeichnet, dass** die Ketoverbindung in einer Menge von 3% bis 50%, bevorzugt von 5% bis 40%, insbesondere von 10%- 30%, bezogen auf das Gesamtvolumen,

eingesetzt wird.

8. Verfahren nach einem der Ansprüche 1 bis 7, **dadurch gekennzeichnet, dass** der TTN (total turn over number = mol Ketoverbindung/ mol eingesetzter Cofactor) $\geq 10^3$ ist.

9. Verfahren nach einem der Ansprüche 1 bis 8, **dadurch gekennzeichnet, dass** es in einem wässrigen organischen Zweiphasensystem durchgeführt wird.

10. Verfahren nach einem der Ansprüche 1 bis 9, **dadurch gekennzeichnet, dass** zusätzlich ein organisches Lösungsmittel, ausgewählt aus Diethylether, tertiär-Butylmethylether, Diisopropylether, Dibutylether, Ethylacetat, Butylacetat, Heptan, Hexan und Cyclohexan, eingesetzt wird.

## Claims

1. Method for stereoselective, in particular enantioselective, enzymatic reduction of keto compounds to the corresponding chiral hydroxy compounds, wherein the keto compounds are reduced by a bacterial enantioselective, NADH-specific oxidoreductase, **characterized in that** reducing the keto compounds involves using a polypeptide which has an R-ADH-signature H-[P; A]-[I; A; Q; V; L]-[G; K]-R at positions 205-209 and the following further structural features in their entirety:

   (i) an N-terminal GxxxGxG Rossmann fold,
   (ii) an NAG-motif at position 87,
   (iii) a catalytic triad consisting of S 139, Y 152 and K 156,
   (iv) a negatively charged amino acid residue at position 37,
   (v) two C-terminal motifs in the dimerization domain [A; S]-S-F and [V; I]-DG-[G; A]-Y-[T; C; L]-[A; T; S]-[Q; V; R; L; P],
   (vi) Val or Leu at position 159,
   (vii) Asn at position 178, and
   (viii) a proline residue at position 188,

   wherein the position of the structural features is determined by Multi-way amino acid comparison using the BLOSUM 62 scoring matrix with the 2bhd_Strex reference sequence with the amino acid sequence

   MHDLTGKNVIITGGARGLGAEAARQAVAAGAHVLITDVLDDDGENAARELGDRARFLHHD
   VTSEEDWSRAADFAVTEFGALHGLVNNAGISTGTPLESESVDHFRKVLDVNLTGVFIGMK
   TVVPALKEAGGGSIVNISSAAGLMGLALTAGYGASKWGVRGLTKIGAVEWGTARVRVNSV
   HPGMTYTPMTAAVGIERGEGKYPNTPMGRVGEADEIAGAVVFLLSDAASYVTGAELAVDG
   GWTTGPTVAYVMGQ

   and the amino acid sequences SEQ ID NO:1 to SEQ ID NO:7.

2. Method according to Claim 1, **characterized in that** a functional oxidoreductase is used for reducing the keto compounds,

   (a) which has any of the amino acid sequences SEQ ID NO:1 to SEQ ID NO:7, or
   (b) in which at least 70 % of the amino acids are identical to the amino acids of any of the amino acid sequences SEQ ID NO:1 to SEQ ID NO:7, or
   (c) which is encoded by a nucleic acid sequence from the group consisting of SEQ ID NO:8 to SEQ ID NO:14, or
   (d) which is encoded by a nucleic acid sequence which hybridizes with one of the nucleic acid sequences mentioned in (c) under stringent conditions.

3. Method according to Claim 1 or 2, **characterized in that** keto compounds of the general formula I are used,

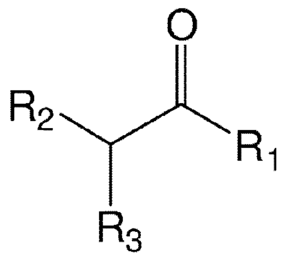

(I)

wherein $R_1$, $R_2$ and $R_3$ are independently selected from the group consisting of:

1) -H, provided that $R_1$ is not H,
2) $-(C_1-C_{20})$alkyl, wherein alkyl is linear-chain or branched,
3) $-(C_2-C_{20})$alkenyl, wherein alkenyl is linear-chain or branched and optionally contains up to four double bonds,
4) $-(C_2-C_{20})$alkynyl, wherein alkynyl is linear-chain or branched and optionally contains up to four triple bonds,
5) $-(C_6-C_{24})$aryl,
6) $-(C_1-C_8)$alkyl-$(C_6-C_{14})$aryl,
7) $-(C_5-C_{14})$heterocycle,
8) $-(C_3-C_7)$cycloalkyl,

wherein the radicals mentioned above under 2) to 8) are unsubstituted or are, independently of one another, mono-, di- or tri-substituted by -OH, halogen, $-NO_2$, $-NH_2$, -NHP and/or -M, wherein P is $-(C_1-C_7)$alkyl, $-(C_2-C_7)$alkenyl, $-(C_2-C_7)$alkynyl, - $(C_6-C_{14})$aryl or a protective group selected from benzyloxycarbonyl, triphenylmethyl and t-butylcarbonyl, and M is $-(C_1-C_7)$alkyl, $-(C_2-C_7)$alkenyl, $-(C_2-C_7)$alkynyl, $-(C_6-C_{14})$aryl or - $(C_1-C_8)$alkyl-$(C_6-C_{14})$aryl, wherein $-(C_6-C_{14})$aryl in $-(C_1-C_8)$alkyl-$(C_6-C_{14})$aryl is unsubstituted or mono-, di- or tri-substituted by halogen, and

9) $-CH_2$-X-R, wherein X is O or S and R is selected from $-(C_1-C_7)$alkyl, phenyl and benzyl, wherein phenyl and benzyl are mono-, di- or tri-substituted by $-(C_1-C_7)$alkyl, $-S(C_1-C_3)$alkyl, $-O(C_1-C_3)$alkyl, $-NO_2$, $-SCF_3$, halogen, $-C(O)(C_1-C_3)$alkyl and/or - CN,

or $R_1$ with $R_2$, $R_1$ with $R_3$, or $R_2$ with $R_3$ form a ring comprising 3-6 carbon atoms which is unsubstituted or, independently of one another, mono-, di- or tri-substituted by -OH, halogen, - $NO_2$, $-NH_2$, -NHP and/or -M, and the remaining radical is selected from the radicals mentioned above under 1) to 9).

**4.** Method according to Claim 1 or 2, **characterized in that** diketones of the general formula II

(II)

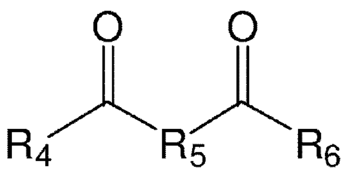

are used as keto compounds, wherein

$R_5$ is $(CH_2)_n$, where n=0-20, $-(C_6-C_{14})$aryl or $-(C_5-C_{14})$-heterocycle,
$R_4$ and $R_6$, independently of one another, are $-(C_1-C_{20})$alkyl or an ester group,
wherein $R_4$, $R_5$ and $R_6$ are unsubstituted or, independently of one another, are mono-, di- or tri-substituted by $-(C_1-C_4)$-alkyl, -OH, halogen, $-NO_2$ and/or $-NH_2$.

**5.** Method according to any of Claims 1 to 4, **characterized in that**

- the oxidized cofactor NAD formed by the oxidoreductase/dehydrogenase is regenerated continuously, and
- a secondary alcohol having the general formula $R_XR_YCHOH$ is used for cofactor regeneration, wherein $R_X$ and $R_Y$, independently of one another, are a branched or unbranched $C_1-C_8$alkyl group and $C_{total}\geq 3$.

6. Method according to Claim 5, **characterized in that** the co-substrate or secondary alcohol used is an alcohol from the group consisting of 2-propanol, 2-butanol, 2-pentanol, 4-methyl-2-pentanol, 2-heptanol and 2-octanol, preferably 2-propanol.

7. Method according to any of Claims 1 to 6, **characterized in that** the keto compound is used in an amount from 3 % to 50 %, preferably from 5 % to 40 %, in particular from 10 % - 30 %, based on the total volume.

8. Method according to any of Claims 1 to 7, **characterized in that** TTN (total turn over number=mol of keto compound/mol of cofactor used) is $\geq 10^3$.

9. Method according to any of Claims 1 to 8, **characterized in that** the method is carried out in an aqueous organic two-phase system.

10. Method according to any of Claims 1 to 9, **characterized in that**, in addition, an organic solvent selected from diethyl ether, tertiary butyl methyl ether, diisopropyl ether, dibutyl ether, ethyl acetate, butyl acetate, heptane, hexane and cyclohexane is used.

## Revendications

1. Procédé de réduction enzymatique stéréosélective, en particulier énantiosélective de composés cétoniques en les composés hydroxy chiraux correspondants, dans lequel les composés cétoniques sont réduits avec une oxydoréductase bactérienne énantiosélective, spécifique de NADH, **caractérisé en ce que** pour la réduction des composés cétoniques, un polypeptide est utilisé, qui présente une signature R-ADH H-[P ; A]-[I ; A ; Q ; V ; L]-[G ; K]-R sur la position 205-209 et d'autres caractéristiques structurelles suivantes dans leur totalité :

   (i) un pli Rossmann GxxxGxG N-terminal,
   (ii) un motif NAG en position 87,
   (iii) une triade catalytique consistant en S 139, Y 152 et K 156,
   (iv) un résidu d'acide aminé à charge négative en position 37,
   (v) deux motifs C-terminaux dans les domaines de dimérisation [A ; S]-S-F et [V ; I]-DG-[G ; A]-Y-[T ; C ; L]-[A ; T ; S]-[Q ; V ; R ; L ; P],
   (vi) Val ou Leu en position 159,
   (vii) Asn en position 178, et
   (viii) un radical de proline en position 188,

   dans lequel la position des caractéristiques structurelles est déterminée par une comparaison multivoie d'acides aminés avec une matrice de notation BLOSUM 62 avec la séquence de référence 2bhd_Strex avec la séquence d'acides aminés :

   MHDLTGKNVIITGGARGLGAEAARQAVAAGAHVLITDVLDDDGENAARELGDRARFLHHD
   VTSEEDWSRAADFAVTEFGALHGLVNNAGISTGTPLESESVDHFRKVLDVNLTGVFIGMK
   TVVPALKEAGGGSIVNISSAAGLMGLALTAGYGASKWGVRGLTKIGAVEWGTARVRVNSV
   HPGMTYTPMTAAVGIERGEGKYPNTPMGRVGEADEIAGAVVFLLSDAASYVTGAELAVDG
   GWTTGPTVAYVMGQ

   et les séquences d'acides aminés SEQ ID NO: 1 à SEQ ID NO: 7.

2. Procédé selon la revendication 1, **caractérisé en ce que**, pour la réduction des composés cétoniques, une oxydoréductase fonctionnelle est utilisée,

   (a) qui présente l'une des séquences d'acides aminés SEQ ID NO: 1 à SEQ ID NO: 7 ou
   (b) dans laquelle au moins 70 % des acides aminés sont identiques aux acides aminés de l'une des séquences d'acides aminés SEQ ID NO: 1 à SEQ ID NO: 7 ou
   (c) pour laquelle code une séquence d'acide nucléique du groupe consistant en SEQ ID NO: 8 à SEQ ID NO: 14 ou

(d) pour laquelle code une séquence d'acide nucléique qui s'hybride avec l'une des séquences d'acide nucléique mentionnées au point (c) dans des conditions stringentes.

**3.** Procédé selon la revendication 1 ou 2, **caractérisé en ce que** des composés cétoniques de formule générale I sont utilisés,

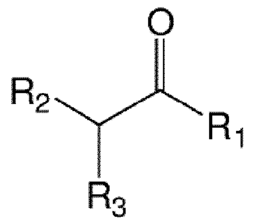

(I)

dans laquelle $R_1$, $R_2$ et $R_3$ sont choisis indépendamment dans le groupe consistant en

1) -H, à condition que $R_1$ ne soit pas H,
2) un groupe alkyle (en $C_1$ à $C_{20}$), où le groupe alkyle est à chaîne linéaire ou ramifiée,
3) un groupe alcényle (en $C_2$ à $C_{20}$), où le groupe alcényle est à chaîne linéaire ou ramifiée et contient éventuellement jusqu'à quatre doubles liaisons,
4) un groupe alcinyle (en $C_2$ à $C_{20}$), où le groupe alcinyle est à chaîne linéaire ou ramifiée et contient éventuellement jusqu'à quatre triples liaisons,
5) un groupe aryle (en $C_6$ à $C_{24}$),
6) un groupe alkyle (en $C_1$ à $C_8$)-aryle (en $C_6$ à $C_{14}$),
7) un hétérocycle (en $C_5$ à $C_{14}$),
8) un groupe cycloalkyle (en $C_3$ à $C_7$),

dans lequel les radicaux mentionnés ci-dessus aux points 2) à

8) sont non substitués ou sont mono-, di- ou tri-substitués indépendamment les uns des autres par -OH, un atome d'halogène, $-NO_2$, $-NH_2$, -NHP et/ou -M, dans lequel P désigne un groupe alkyle (en $C_1$ à $C_7$), alcényle (en $C_2$ à $C_7$), alcinyle (en $C_2$ à $C_7$), aryle (en $C_6$ à $C_{14}$) ou un groupe protecteur choisi parmi les groupes benzyloxycarbonyle, triphénylméthyle et t-butylcarbonyle et M désigne un groupe alkyle (en $C_1$ à $C_7$), alcényle (en $C_2$ à $C_7$), alcinyle (en $C_2$ à $C_7$), aryle (en $C_6$ à $C_{14}$) ou alkyle (en $C_1$ à $C_8$)-aryle (en $C_6$ à $C_{14}$), dans lequel le groupe aryle (en $C_6$ à $C_{14}$) dans le groupe alkyle (en $C_1$ à $C_8$)-aryle (en $C_6$ à $C_{14}$) est non substitué ou mono-, di- ou tri-substitué par un atome d'halogène, et
9) $-CH_2$-X-R, dans lequel X désigne O ou S et R est choisi parmi les groupes alkyle (en $C_1$ à $C_7$), phényle et benzyle, dans lequel les groupes phényle et benzyle sont mono-, di- ou tri-substitués par un groupe alkyle (en $C_1$ à $C_7$), -S-alkyle (en $C_1$ à $C_3$), -O(alkyle en $C_1$ à $C_3$), $-NO_2$, $-SCF_3$, un atome d'halogène, -C(O)-alkyle (en $C_1$ à $C_3$) et/ou -CN,

ou $R_1$ avec $R_2$, $R_1$ avec $R_3$ ou $R_2$ avec $R_3$ forment un cycle de 3 à 6 atomes de carbone qui est non substitué ou mono-, di- ou tri-substitué indépendamment les uns des autres par -OH, un atome d'halogène, $-NO_2$, $-NH_2$, -NHP et/ou -M et le radical restant est choisi parmi les radicaux mentionnés aux points 1) à 9).

**4.** Procédé selon la revendication 1 ou 2, **caractérisé en ce que** comme composés cétoniques, des dicétones de formule générale II sont utilisées

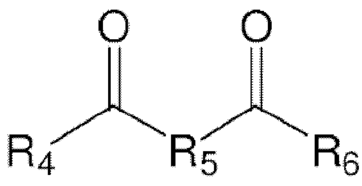

(II)

dans laquelle

$R_5$ désigne $(CH_2)_n$ avec n = 0 à 20, un groupe aryle (en $C_6$ à $C_{14}$), ou un hétérocycle (en $C_5$ à $C_{14}$), $R_4$ et $R_6$ désignent indépendamment les uns des autres, un groupe alkyle (en $C_1$ à $C_{20}$) ou un groupe ester, dans lequel $R_4$, $R_5$ et $R_6$ sont non substitués ou sont mono-, di-, ou tri-substitués indépendamment les uns des autres par un groupe alkyle (en $C_1$ à $C_4$), -OH, un atome d'halogène, $-NO_2$ et/ou $-NH_2$.

**5.** Procédé selon l'une des revendications 1 à 4, **caractérisé en ce que**

- le co-facteur NAD oxydé, formé par l'oxydoréductase/déshydrogénase est régénéré en continu, et
- pour la régénération du co-facteur, un alcool secondaire de formule générale $R_XR_YCHOH$ est utilisé, dans lequel $R_X$ et $R_Y$ sont indépendamment l'un de l'autre un groupe alkyle en $C_1$ à $C_8$ ramifié ou non ramifié et $C_{total} \geq 3$.

**6.** Procédé selon la revendication 5, **caractérisé en ce que** comme co-substrat ou comme alcool secondaire, un alcool du groupe consistant en 2-propanol, 2-butanol, 2-pentanol, 4-méthyl-2-pentanol, 2-heptanol et 2-octanol, de préférence 2-propanol est utilisé.

**7.** Procédé selon l'une des revendications 1 à 6, **caractérisé en ce que** le composé cétonique est utilisé en une quantité de 3 % à 50 %, de préférence de 5 % à 40 %, en particulier de 10 % à 30 %, par rapport au volume total.

**8.** Procédé selon l'une des revendications 1 à 7, **caractérisé en ce que** le TTN (total turn over number = mole de composé cétonique/mole de cofacteur utilisé) $\geq 10^3$.

**9.** Procédé selon l'une des revendications 1 à 8, **caractérisé en ce qu'**il est réalisé dans un système diphasique aqueux organique.

**10.** Procédé selon l'une des revendications 1 à 9, **caractérisé en ce qu'**un solvant organique choisi parmi le diéthyléther, le butylméthyléther tertiaire, le diisopropyléther, le dibutyléther, l'acétate d'éthyle, l'acétate de butyle, l'heptane, l'hexane et le cyclohexane est en outre utilisé.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1383899 A
- WO 2006087235 A
- WO 2007036257 A
- US 5523223 A
- US 5763236 A
- DE 10327454
- WO 2007012428 A
- US 5200335 A
- DE 19610984 A1
- DE 10119274
- EP 1152054 A1
- WO 2007033928 A
- WO 2006046455 A
- WO 2005033094 A
- EP 1179595 A1
- US 7172894 B2
- WO 2004027055 A


### In der Beschreibung aufgeführte Nicht-Patentliteratur


- *Enzyme Microb. Technol.,* November 1993, vol. 15 (11), 950-8
- *Biosci. Biotechnol. Biochem.,* 1999, vol. 63 (10), 1721-1729
- *Appl. Microbiol. Biotechnol.,* September 2003, vol. 62 (4), 380-6
- *J. Org. Chem.,* 24. Januar 2003, vol. 68 (2), 402-6
- *Acta Crystallogr. D Biol. Crystallogr.,* Dezember 2000, vol. 56, 1696-8
- *Biosci. Biotechnol. Biochem.,* 2006, vol. 70 (2), 418-426
- **ALTSCHUL et al.** *Proc. Natl. Acd. Sei. USA,* 1990, vol. 87, 2264-2268
- A model of evolutionary change in Proteins. **DAYHOFF; M.O. ; SCHWARZ, R.M. ; ORCUTT, B.C.** Atlas of Protein Sequence and structure. National Biomedical Research foundation, 1978, vol. 5, 345-352
- Protokol 32 von Molecular Cloning, A Laboratory Manual. Cold Spring Harbor Laboratory Press, 1989

SEQUENCE LISTING

<110> IEP GmbH

<120> Prozess zur stereoselektiven Reduktion von Ketoverbindungen

<130> I 13889

<160> 14

<170> PatentIn version 3.3

<210> 1
<211> 247
<212> PRT
<213> Bacillus cereus

<400> 1

```
Met Lys Leu Lys Asp Lys Val Ala Ile Ile Thr Gly Gly Ala Ser Gly
1               5                   10                  15

Ile Gly Glu Ser Thr Val Arg Leu Phe Ile Glu Glu Gly Ala Lys Val
            20                  25                  30

Val Ile Ala Asp Phe Ser Glu Arg Gly Lys Glu Leu Ser Asp Glu Leu
        35                  40                  45

Asn Ala His Gly Tyr Asn Thr Leu Phe Ile Lys Thr Asp Val Thr Lys
    50                  55                  60

Glu Ala Asp Ile Lys Gln Leu Ile His Glu Thr Val Ser Thr Tyr Gly
65                  70                  75                  80

Lys Leu Asp Ile Met Tyr Ala Asn Ala Gly Val Ala Asp Asp Ala Pro
                85                  90                  95

Ala Asn Glu Leu Ser Tyr Glu Lys Trp Lys Arg Thr Ile Asp Ile Asn
            100                 105                 110

Leu Ser Gly Val Phe Leu Ser Asp Lys Tyr Ser Ile Glu Gln Phe Leu
        115                 120                 125

Lys Gln Gly Thr Gly Gly Val Ile Val Asn Ala Gly Ser Ile His Ser
    130                 135                 140

Phe Val Ser Leu Pro Thr Pro Thr Ala Tyr Ser Ser Ala Lys Gly Gly
145                 150                 155                 160

Val Lys Leu Leu Thr Gln Asn Leu Cys Thr Ala Tyr Ala Lys Tyr Gly
```

165 170 175

Ile Arg Ile Asn Ala Val Cys Pro Gly Tyr Ile Asp Thr Pro Leu Leu
180 185 190

Gly Ser Val Asn Pro Gln Gln Lys Glu Tyr Leu Ala Ser Leu His Pro
195 200 205

Gln Gly Arg Leu Gly Thr Pro Glu Glu Val Ala Lys Ala Val Leu Phe
210 215 220

Leu Ala Ser Asp Asp Ala Ser Phe Val Asn Gly Thr Thr Leu Leu Val
225 230 235 240

Asp Gly Gly Tyr Thr Ala Arg
245

<210> 2
<211> 257
<212> PRT
<213> Methylibium petroleiphilum

<400> 2

```
Met Gly Arg Val Asp Asn Lys Val Ala Leu Val Thr Gly Gly Ala Lys
1               5                   10                  15

Gly Ile Gly Arg Ala Ser Ala Leu Met Leu Ala Arg Glu Gly Ala Arg
            20                  25                  30

Val Val Leu Thr Asp Val Glu Glu Ala Gln Gly Ser Ala Val Ala Lys
        35                  40                  45

Glu Ile Glu Arg Ala Gly Gly Lys Ala Leu Phe Leu Thr Gln Asp Val
    50                  55                  60

Thr Asp Glu Ser Arg Trp Val Glu Val Val Glu Lys Ala Arg Ala Gln
65                  70                  75                  80

Phe Gly Gly Leu Asn Ile Val Val Asn Asn Ala Gly Ile Gly Thr Ala
                85                  90                  95

Gly Ser Ala Glu Asp Glu Thr Leu Glu Ala Trp Arg Arg Leu Met Ser
            100                 105                 110

Val Asn Leu Asp Gly Val Phe Leu Gly Thr Lys His Ala Ile Arg Ala
        115                 120                 125
```

```
Met Lys Asn Gly Pro Gly Thr Gly Ser Ile Ile Asn Ile Ser Ser Ile
    130                 135                 140

Glu Gly Ile Val Ala Asp Pro Lys Leu Ala Ser Tyr Asn Ala Ser Lys
145                 150                 155                 160

Gly Gly Val Arg Ile Phe Ser Lys Ser Ala Ala Leu His Cys Ala Gln
                165                 170                 175

Ala Gly Tyr Arg Ile Arg Val Asn Thr Ile His Pro Gly Tyr Ile Trp
            180                 185                 190

Thr Pro Met Val Glu Gly Tyr Leu Thr Ser Leu Gly Asp Val Glu Gly
        195                 200                 205

Gly Arg Gln Val Ile Ser Lys Met His Pro Ile Gly Arg Met Gly Glu
    210                 215                 220

Pro Asp Asp Ile Ala Tyr Gly Val Leu Tyr Leu Gly Ser Asp Glu Ser
225                 230                 235                 240

Ser Phe Met Thr Gly Ser Glu Leu Val Ile Asp Gly Gly Tyr Thr Ala
                245                 250                 255

Gln
```

<210> 3
<211> 251
<212> PRT
<213> Mezorhizobium sp.

<400> 3

```
Met Thr Gly Glu Phe Lys Asp Lys Val Ala Leu Val Thr Gly Ala Gly
1               5                   10                  15

Ser Gly Ile Gly Ala Ala Ile Ala Arg Glu Leu Ala Thr Gly Gly Ala
            20                  25                  30

Glu Leu Val Val Ala Asp Leu Glu Arg Glu Ser Ala Asn Arg Ile Val
        35                  40                  45

Glu Gly Ile Arg Ala Ser Gly Gly Arg Ala His Ala Phe Ala Val Asp
    50                  55                  60
```

```
Val Ala Asp Ala Glu Ala Val Glu Arg Met Val Asp Phe Ala Val Arg
65                  70                  75                  80

Thr Cys Gly Asp Leu His Leu Ala Val Asn Asn Ala Gly Ile Gly Gly
                85                  90                  95

Pro Ser Glu Pro Thr Ala Asp Tyr Pro Leu Asp Gly Trp Arg Lys Val
            100                 105                 110

Ile Asp Val Asn Leu Asn Gly Val Phe Tyr Gly Met Lys Tyr Glu Ile
        115                 120                 125

Ala Ala Ile Leu Lys Ser Gly Gly Gly Ala Ile Val Asn Met Ala Ser
    130                 135                 140

Ile Leu Gly Ser Val Gly Phe Ala Asn Ala Cys Ala Tyr Val Ser Ala
145                 150                 155                 160

Lys His Ala Leu Leu Gly Leu Thr Lys Thr Ala Ala Met Glu Tyr Ala
                165                 170                 175

Ala Gln Gly Val Arg Ile Asn Ala Val Gly Pro Ala Phe Ile Asp Thr
            180                 185                 190

Pro Leu Leu Ser Lys Asn Leu Asp Asp Gln Val Leu Gly Gln Leu Ala
        195                 200                 205

Gly Leu His Pro Ile Gly Arg Leu Gly Thr Pro Glu Glu Val Ser Ala
    210                 215                 220

Leu Thr Cys Phe Leu Leu Ser Gly Arg Ala Ser Phe Ile Thr Gly Ser
225                 230                 235                 240

Tyr His Leu Val Asp Gly Gly Tyr Thr Thr Arg
                245                 250
```

<210> 4
<211> 263
<212> PRT
<213> Streptomyces coelicolor

<400> 4

```
Met Ser Thr Thr Gly Thr Thr Pro Ala Thr Thr Gly Tyr Ala Ala Glu
1               5                   10                  15
```

```
Phe Ala Gly Arg Thr Ala Leu Val Thr Gly Ala Ala Ser Gly Ile Gly
            20                  25                  30

Leu Ala Thr Ala Arg Arg Leu Gly Ala Gly Gly Ala Arg Val Val Val
        35                  40                  45

Ala Asp Phe Asn Ala Glu Gly Ala Glu Lys Ala Ala Ala Glu Leu Arg
    50                  55                  60

Ala Gly Gly Val Glu Ala Ala Ala Val Glu Leu Asp Val Thr Arg Pro
65                  70                  75                  80

Glu Ser Val Glu Ala Ala Val Gly Phe Ala Val Asp Thr Phe Gly Ser
                85                  90                  95

Leu Asp Leu Ala Val Asn Asn Ala Gly Ile Gly Gly Pro Ser Ala Pro
            100                 105                 110

Thr Gly Glu Tyr Asp Val Ala Ala Tyr Gln Arg Val Val Arg Thr Asn
        115                 120                 125

Leu Asp Gly Val Phe Tyr Ser Met Arg Tyr Glu Leu Pro Ala Ile Glu
    130                 135                 140

Ala Ala Gly Lys Gly Gly Ser Ile Val Asn Val Ala Ser Ile Leu Gly
145                 150                 155                 160

Ser Val Gly Phe Ala Gly Ser Pro Ala Tyr Val Ala Ala Lys His Gly
                165                 170                 175

Val Val Gly Leu Thr Lys Ala Ala Ala Ala Glu Tyr Ala Ala Arg Gly
            180                 185                 190

Ile Arg Ile Asn Ala Val Gly Pro Gly Phe Ile Asp Thr Pro Leu Leu
        195                 200                 205

Lys Thr Met Asp Glu Ala Ala Tyr Lys Gly Leu Val Ala Leu His Pro
    210                 215                 220

Ala Gly Arg Leu Gly Arg Ser Glu Glu Val Ala Glu Leu Ile Ala Phe
225                 230                 235                 240

Leu Leu Ser Asp Arg Ala Ser Phe Val Ala Gly Ser Tyr His Leu Val
                245                 250                 255
```

Asp Gly Ala Tyr Thr Ala Val
260

<210> 5
<211> 253
<212> PRT
<213> Ralstonia eutropha

<400> 5

```
Met Glu Tyr Ile Asp Met Lys Leu Lys Asp Lys Val Ala Ile Val Thr
1               5                   10                  15

Gly Gly Ala Ser Gly Ile Gly Glu Ala Thr Val Arg Leu Phe Ala Ser
            20                  25                  30

Gln Gly Ala Ser Val Val Ile Ala Asp Arg Ser Ala Leu Gly Glu Lys
        35                  40                  45

Leu Ala Arg Glu Leu Ser Glu Ser Ser Leu Ala Ala His Tyr Ser Glu
    50                  55                  60

Val Asp Val Ser Arg Glu Asp Asp Thr Arg Arg Leu Ile Asp Asp Thr
65                  70                  75                  80

Val Ser Arg Phe Gly Arg Leu Asp Ile Met Val Ala Asn Ala Gly Ile
                85                  90                  95

Ala His Pro Ser Ala Pro Val Glu Asp Val Ser Val Glu Gln Trp Gln
            100                 105                 110

Gln Met Ile Asp Val Asn Leu Thr Gly Val Phe Leu Ser Asn Lys Leu
        115                 120                 125

Ala Ile Val Gln Met Lys Lys Gln Gly Thr Gly Gly Ala Ile Val Asn
    130                 135                 140

Met Ala Ser Ile Leu Gly His Val Gly Met Pro Gly Ala Ala Ser Tyr
145                 150                 155                 160

Asn Ala Ala Lys Gly Gly Val Val Asn Leu Thr Arg Ser Leu Gly Val
                165                 170                 175

Ser His Ala Gln Asp Gly Ile Arg Val Asn Ala Val Cys Pro Gly Phe
            180                 185                 190

Val Ala Thr Pro Leu Ile Glu Arg Ala Thr Glu Glu Ala Arg Ala Arg
```

```
            195                 200                 205

Leu Val Ala Ala His Pro Ile Gly Arg Leu Gly His Ala Asp Glu Val
    210                 215                 220

Ala Lys Ala Val Leu Phe Leu Ala Ser Asp Asp Ala Ser Phe Ile Val
225                 230                 235                 240

Gly Thr Ser Leu Met Val Asp Gly Gly Tyr Cys Ala Gln
                245                 250
```

<210> 6
<211> 252
<212> PRT
<213> Herpentosiphon aurantiacus

<400> 6

```
Met Asn Gln Tyr Asp Phe Arg Gly Lys Val Ala Leu Val Thr Gly Gly
1               5                   10                  15

Ala Ser Gly Ile Gly Ala Ala Cys Val His Thr Phe Ala Arg Gly Gly
            20                  25                  30

Ala Lys Val Ala Ile Val Asp Arg Asn Gln Asp Leu Gly Ala Gln Thr
        35                  40                  45

Val Ala Ala Val Arg Glu Ala Gly Gly Asp Ala Ile Phe Leu Pro Val
    50                  55                  60

Asp Val Ala Gln Ser Gly Ala Val Glu Ala Met Val Thr Asp Thr Ile
65                  70                  75                  80

Thr Ala Phe Gly Gln Leu Asp Ile Ala Val Asn Asn Ala Gly Ile Gly
                85                  90                  95

Gly Glu Ser Asn Pro Thr Gly Thr Tyr Ser Ile Glu Gly Trp Gln Thr
            100                 105                 110

Val Ile Asp Val Asn Leu Asn Gly Val Phe Tyr Cys Met Arg Tyr Glu
        115                 120                 125

Ile Pro Ala Met Leu Ala Gln Gly Gly Gly Val Ile Val Asn Met Ala
    130                 135                 140

Ser Ile Leu Gly Thr Val Gly Phe Ala Ser Ser Pro Ala Tyr Val Ala
145                 150                 155                 160
```

Ala Lys His Ala Val Val Gly Leu Thr Lys Ala Ala Ala Leu Asp Tyr
165 170 175

Gly Arg Gln Gly Leu Arg Ile Asn Ser Val Gly Pro Gly Phe Ile Lys
180 185 190

Thr Pro Leu Leu Asp Gly Gly Leu Asp Asp Gln Thr Gln Thr Tyr Leu
195 200 205

Ser Gly Leu His Ala Val Gly Arg Met Gly Glu Ser Ala Glu Val Ala
210 215 220

Ala Leu Val Ala Phe Leu Cys Ser Ala Glu Ala Ser Phe Leu Thr Gly
225 230 235 240

Gly Tyr Tyr Leu Val Asp Gly Gly Tyr Thr Ala Gln
245 250

<210> 7
<211> 249
<212> PRT
<213> Paracoccus denitrificans

<400> 7

```
Met Asp Ile Arg Phe Asp Asn Lys Ile Ala Leu Val Thr Gly Ala Gly
1               5                   10                  15

Ser Gly Leu Gly Glu Ala Ile Ala Leu Glu Leu Ala Ala Ser Gly Ala
            20                  25                  30

Thr Val Val Ala Ala Asp Leu His Glu Ala Thr Ala Arg Ala Thr Ala
        35                  40                  45

Asp Arg Ile Val Ala Ala Gly Gly Lys Ala Lys Ala Val Ala Gly Asp
    50                  55                  60

Val Ser Asp Pro Asp Ala Val Arg Lys Ala Val Glu Val Ala Lys Gly
65                  70                  75                  80

Leu Gly Gly Leu His Leu Leu Val Asn Asn Ala Gly Ile Gly Gly Pro
                85                  90                  95

Ser Ala Pro Val Gly Asp Tyr Pro Leu Asp Gly Trp Lys Lys Val Ile
            100                 105                 110
```

```
Asp Val Asn Leu Asn Ala Val Phe Tyr Gly Met Arg Tyr Gly Ile Pro
        115                 120                 125

Ala Met Leu Asp Ala Gly Gly Gly Ala Ile Val Asn Met Ala Ser Ile
    130                 135                 140

Leu Gly Ser Val Gly Phe Asn Gly Ala Gly Ala Tyr Val Ser Ala Lys
145                 150                 155                 160

His Ala Val Val Gly Met Thr Lys Asn Ala Ala Leu Glu Tyr Ala Gln
                165                 170                 175

Lys Gly Ile Arg Val Asn Ser Val Gly Pro Ala Phe Ile Asp Thr Pro
            180                 185                 190

Leu Leu Asp Gln Leu Asp Ser Asp Thr Arg Gln Ala Leu Val Gly Arg
        195                 200                 205

His Pro Ile Gly Arg Leu Gly Arg Ala Asp Glu Val Ala Gly Leu Val
    210                 215                 220

Val Phe Leu Leu Ser Asp Arg Ala Ser Phe Ile Thr Gly Ser Tyr His
225                 230                 235                 240

Leu Val Asp Gly Gly Tyr Thr Ala Leu
                245
```

<210> 8
<211> 744
<212> DNA
<213> Bacillus cereus

<400> 8

atgaaattaa aagacaaagt agcaatcata actggtggtg caagtggaat tggcgaatct 60
actgttcgtc tttttatcga agaaggtgca aaagtagtta ttgctgactt ttctgaacgt 120
ggaaaagaac tatcagatga attgaatgca catggatata atacgttatt tattaaaacc 180
gatgtaacaa aagaggcaga tattaaacag ctaattcatg agacagtaag tacatacggt 240
aaattagata ttatgtatgc caatgccggc gttgctgatg atgcaccggc aaacgaatta 300
tcctatgaaa agtggaaaag aactattgat attaatttgt ctggggtatt cctttctgat 360
aaatattcga ttgaacaatt tcttaaacaa ggtacaggtg gtgtcatcgt taacgctggt 420
tcgattcata gttttgtttc attacctaca ccaacagcat actcctctgc aaaaggtggt 480
gtgaaactat taactcaaaa tttatgtact gcctacgcta aatatggaat acgtattaac 540

gcggtgtgcc ctggttatat tgacacccct ttactaggta gtgttaatcc tcaacagaaa 600
gaatatttag cttcacttca tccacaaggc agacttggaa caccagaaga agtcgctaaa 660
gctgtcttat ttttagcaag tgatgatgct agttttgtta acggcacaac acttcttgtt 720
gatggaggct atactgcacg ttaa 744

<210> 9
<211> 774
<212> DNA
<213> Methylibium petroleiphilum

<400> 9

```
atgggacgtg tggacaacaa ggtggcgctg gtgaccggtg gtgccaaggg catcggccgg      60
gccagcgcac tgatgctggc gcgcgaaggt gcccgcgtgg tgctgaccga cgtggaagag     120
gcgcagggca gcgcggtggc gaaggagatc gagcgcgccg gcggcaaggc gctgttcctc     180
acgcaggacg tgaccgacga gagccgctgg gtcgaggtgg tcgagaaggc ccgcgcgcag     240
ttcggcggcc tgaacatcgt cgtcaacaac gccggcatcg gcaccgctgg cagcgccgag     300
gacgagacgc tggaggcctg gcgccgcctg atgtccgtca acctcgacgg cgtcttcctc     360
ggcaccaagc acgcgatccg ggcgatgaag aacgggcccg gcacgggctc gatcatcaac     420
atctcgtcga tcgagggcat cgtcgccgac cccaagctcg cgtcctacaa cgccagcaag     480
ggcggcgtgc gcatcttctc gaagtcggcg gcactgcatt gcgcgcaggc gggctaccgc     540
atccgggtta acaccatcca tccgggctac atctggacgc cgatggtcga aggctatctg     600
accagcctcg gcgatgtcga gggtgggcgc caggtcatct cgaagatgca cccgatcggg     660
cggatgggtg agcccgacga catcgcctac ggtgtgctct acctgggctc cgacgagtcc     720
tcgttcatga cgggcagcga gctggtcatc gacggcggct acaccgcgca atag           774
```

<210> 10
<211> 756
<212> DNA
<213> Mesorhizobium sp.

<400> 10

```
atgactggtg aattcaagga caaggttgcg ctcgtcacag gagccggctc ggggataggg      60
gccgcaatcg ctcgcgaact ggccacagga ggcgccgagc tggtcgtcgc cgacctggag     120
cgggagtctg cgaacaggat cgtggaagga atccgagcga gtggaggccg agcccacgcc     180
tttgccgtgg atgtagccga cgccgaggcg gtcgagcgca tggtggattt tgccgttcgc     240
acgtgcggtg accttcatct ggcggtcaat aatgctggga tcggcggccc aagcgaaccg     300
accgccgact atccgctcga cggctggcgc aaggtgatcg atgtcaatct gaatggcgtc     360
```

ttctatggca tgaaatatga gatcgcagcg atactgaaat ccggcggcgg cgccatcgtg 420

aacatggcat ccatcctcgg ttcagtggga tttgcgaatg cctgcgccta tgtctccgcg 480

aaacacgcgc tgctgggact gacaaaaacg gcggcgatgg agtatgcggc acaaggcgtg 540

cgcatcaatg cggtcgggcc agctttcatc gacacgccgc ttctgtcgaa aaacctcgac 600

gaccaagtcc tcggccagct cgcgggactt catcccatcg gccggctcgg cacgcctgag 660

gaggtctcgg cgctcacctg tttcttgctt tccggacgcg cgagcttcat caccggcagt 720

tatcatctcg tcgatggcgg ctacaccacc cggtaa 756

<210> 11
<211> 792
<212> DNA
<213> Streptomyces coelicolor

<400> 11

atgagcacca ccggaaccac ccccgccacc accgggtacg ccgccgagtt cgccggccgt 60

accgccctcg tcaccggtgc cgcctccggt atcggcctgg ccaccgcccg ccggctcggc 120

gccggcggcg cccgggtcgt cgtcgccgac ttcaacgccg agggcgccga gaaggccgcc 180

gccgagctgc gggccggtgg cgtcgaggcc gccgcggtcg agctggacgt cacccgtccg 240

gagtccgtcg aggcggccgt cgggttcgcc gtcgacacgt tcggctcgct ggacctcgcc 300

gtcaacaacg ccggcatcgg cggccccagc gccccgaccg gcgagtacga cgtggcggcc 360

taccagcgcg tcgtgcgcac caacctcgac ggcgtcttct actcgatgcg ctacgaactg 420

cccgccatcg aggcggccgg caagggcggc tcgatcgtga acgtcgcctc catcctcggc 480

tcggtcggct tcgccggctc cccgcctac gtcgccgcca agcacggcgt ggtcgggctg 540

acgaaggcgg ccgccgccga gtacgccgcc cgcggcatcc ggatcaacgc ggtcggtccg 600

ggcttcatcg acaccccct gctcaagacc atggacgagg ccgcctacaa ggggctggtc 660

gccctgcacc cggccggccg cctcgggcgc tccgaggagg tcgcggagct gatcgccttc 720

ctgctgtccg accgcgcgtc cttcgtcgcg ggcagctatc acctggtcga cggcgcctac 780

accgccgtct ga 792

<210> 12
<211> 762
<212> DNA
<213> Ralstonia eutropha

<400> 12

```
atggagtaca tcgacatgaa gctcaaggac aaggtcgcca tcgtaacggg cggtgccagc      60
ggtattggcg aggcaacggt tcggctgttc gcttcccaag gtgccagcgt cgtcattgcc     120
gaccgctcgg cactcggcga gaagctcgcg cgtgaactga gcgaaagcag cttggccgcg     180
cactacagcg aagtcgatgt gtcgcgcgag gacgacacgc gcaggctcat cgatgacacc     240
gtgagccgct tcgggcggct cgacatcatg gtcgccaatg ccggcattgc gcatccgtct     300
gcaccagtcg aggatgtcag tgtcgagcaa tggcagcaga tgatcgacgt caacctgacc     360
ggcgtcttcc tgagcaacaa gcttgccatt gtgcagatga agaagcaggg caccggcggc     420
gcgatcgtca acatggcatc gatactcggg catgtcggga tgccgggcgc ggcgtcctac     480
aacgcggcaa agggcggcgt ggtcaacctc acgcgctctc tcggcgtctc gcatgcacag     540
gacggcatac gcgtcaacgc ggtatgtccg ggtttcgtgg caacaccgct gatcgaacgt     600
gccactgagg aagcccgtgc gcgcctggtg gcggcgcatc cgattggccg cctgggtcac     660
gccgatgagg tcgcgaaggc cgtgctgttt ctcgcgagcg acgatgcatc gttcatcgtc     720
ggtacgagcc tgatggtcga tggagggtat tgcgcgcaat ga                        762
```

<210> 13
<211> 759
<212> DNA
<213> Herpentosiphon aurantiacus

<400> 13

atgaaccagt atgactttcg tggaaaagtg gcgcttgtca ccggcggcgc ctcggggatt 60

ggtgcggcgt gcgtgcatac ctttgcgcgt ggcggcgcga aggtcgcgat tgttgatcgc 120

aatcaggatc tgggcgcaca aaccgtggcg gcggtccgcg aggccggcgg cgatgcgatc 180

tttctcccgg tggatgtcgc ccaatcaggg gccgttgagg cgatggttac cgacaccatc 240

acggcctttg gacagctgga tattgcggtc aataatgccg ggatcggcgg cgagtctaat 300

cctaccggca cctacagcat tgaaggttgg cagacggtca ttgatgtgaa cctcaacggt 360

gtcttctatt gcatgcgcta cgaaattcca gccatgctgg cgcagggcgg cggcgtgatc 420

gtgaatatgg cctcgatcct agggactgtc ggatttgcgt cctccccagc ctatgtcgcc 480

gccaagcacg cggtggttgg tctcaccaag gccgccgccc tggactatgg gcgccaaggg 540

ctgcggatca actcggtcgg gccgggcttt atcaaaacac cactgcttga cggtggcctg 600

gacgatcaga cccaaaccta tctgagcggg ctccacgccg tcggacgcat gggcgagtca 660

gcggaagtcg cggcgctggt cgcctttctc tgctccgctg aggcctcatt cctgacgggt 720

ggctattatc tggtcgatgg tggatacacg gcgcaataa 759

<210> 14
<211> 750
<212> DNA
<213> Paracoccus denitrificans

<400> 14

atggacattc gtttcgacaa caagatcgcc cttgtgaccg gcgccggctc ggggctgggc 60

gaggcgatag cgctggagct tgccgcctcg ggcgccacgg tggtggccgc cgacctgcat 120

gaagcgacag cccgcgccac cgccgacagg atcgtcgcgg cgggcggcaa ggccaaggcc 180

gtggcgggcg atgtctccga tcctgatgcg gtgcgcaagg ccgtcgaggt cgccaagggg 240

ctgggcgggc tgcacctgct ggtgaacaat gccggcatcg gcggccccag cgcgccggtc 300

ggggactatc cgctggacgg ctggaaaaag gtcatcgacg tcaacctgaa cgcggtcttc 360

tacggcatgc gctacgggat tccggcgatg ctggacgcgg gcggcggggc gatcgtgaac 420

atggcctcga tcctgggctc ggtcgggttc aacggcgcgg gcgcctatgt ctcggccaag 480

catgccgtgg tgggcatgac caagaacgcg gcgctggaat atgcgcaaaa gggcatccgg 540

gtgaactcgg tcggcccggc cttcatcgac acgccgctgc tcgaccagtt ggacagcgac 600

accaggcagg cgctggtcgg ccgccatccc atcggccggc tgggccgggc ggacgaggtt 660

gcggggcttg tcgtgttcct gctttcggac cgcgccagct tcatcaccgg cagctatcac 720

ctggtggatg gcggctatac ggcgctgtga 750